(19)

**Europäisches Patentamt**
**European Patent Office**
**Office européen des brevets**

(11)  **EP 4 289 971 A1**

(12)  **EUROPEAN PATENT APPLICATION**

(43) Date of publication:
**13.12.2023  Bulletin 2023/50**

(21) Application number: **22177666.9**

(22) Date of filing: **07.06.2022**

(51) International Patent Classification (IPC):
**C12Q 1/6886** (2018.01)

(52) Cooperative Patent Classification (CPC):
**C12Q 1/6886**; C12Q 2600/154; C12Q 2600/156

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Heinrich-Heine-Universität Düsseldorf 40225 Düsseldorf (DE)**

(72) Inventors:
• **Santourlidis, Simeon 40670 Neuss (DE)**

• **Erichsen, Lars 47623 Kevelaer (DE)**

(74) Representative: **Cohausz & Florack Patent- & Rechtsanwälte Partnerschaftsgesellschaft mbB Bleichstraße 14 40211 Düsseldorf (DE)**

<u>Remarks:</u>
The complete document including Reference Table(s) and the Sequence Listing(s) can be downloaded from the EPO website

(54)  **METHYLATION-SPECIFIC PRIMERS FOR DIAGNOSING TUMORS**

(57)   The invention is directed to a methylation-specific primer for amplifying one or more CpG dinucleotides comprised in SEQ ID NO: 1. The invention is also directed to an *in vitro* method for diagnosing, prognosing, and/or monitoring a tumor, comprising determining the level of methylation of one or more CpG dinucleotides comprised in one of SEQ ID NO: 1-9 of a test sample; determining the level of methylation of the same one or more CpG dinucleotides comprised in one of SEQ ID NO: 1-9 of a control sample; and comparing the level of methylation of the one or more CpG dinucleotides of the test sample to that of the control sample; wherein a hypermethylation in the one or more CpG dinucleotides of the test sample indicates the presence of a tumor, or indicates a high likelihood of developing a tumor.

<u>Figure 4D</u>

EP 4 289 971 A1

**Description**

**FIELD OF THE INVENTION**

The present invention relates to methylation-specific primers, primer pairs, as well as an *in vitro* methods of diagnosing, prognosing and/or monitoring a tumor, in particular in bladder or prostate cancer.

**BACKGROUND OF THE INVENTION**

In 85 to 90% of all human cancers, telomerase activity is observed, contributing limitless self-renewal capacity, although those malignant cells for the most part retain short telomeres. Telomerase activation is most commonly achieved by somatic mutations in the proximal promoter region of the *hTERT* gene, which are among the most prevalent mutations in human cancers or sometimes by proviral insertion. In differentiated somatic cells, *hTERT* expression is absent. In contrast, upregulation of *hTERT* is observed in 85-90% of all human cancers conferring limitless self-renewal capacity and is involved in tumor initiation and progression.

In recent studies, diverse new epigenetic modes of *hTERT* regulation have been reported (Lee DD et al (2019) DNA hypermethylation within TERT promoter upregulates TERT expression in cancer. The Journal of clinical investigation 129: 223-229; Malhotra S, et al (2017) A Novel Long Non-Coding RNA in the hTERT Promoter Region Regulates hTERT Expression. Non-coding RNA 4:10). In these studies a differentially methylated region located slightly upstream of the *hTERT* core promoter region (Chr 5: 1,295,321-1,295,753, GRCh37/hg19; 433 bp, 52 CpG) has been identified to be of regulatory importance for hTERT expression, in particular in cancer. This region is referred to as "*TERT* hypermethylated oncological region" (THOR) and been reported to be hypermethylated in hTERT-expressing pediatric tumors (Castelo-Branco et al (2013) Methylation of the TERT promoter and risk stratification of childhood brain tumours: an integrative genomic and molecular study. Lancet Oncol. 14: 534-42; Castelo-Branco et al (2016) A Cancer Specific Hypermethylation Signature of the TERT Promoter Predicts Biochemical Relapse in Prostate Cancer: A Retrospective Cohort Study. Oncotarget 7: 57726-57736). The THOR region is located within the *hTERT* promoter region and encompasses 433bp with 52 CpG sites. Hypermethylation of this CpG-dense region has been shown to be responsible for upregulation of *hTERT* expression in cancer, while unmethylation of THOR represses *hTERT* promoter activity.

Due to its high prevalence of >45% in all cancer types screened, THOR hypermethylation has been suggested to be a frequent telomerase-activating mechanism in *hTERT*-expressing tumor types, e.g. in cancers of the prostate, breast, blood, colon, skin, bladder and brain (Lee DD et al (2019) DNA hypermethylation within TERT promoter upregulates TERT expression in cancer. The Journal of clinical investigation 129: 223-229). Importantly, in urothelial bladder cancer, where risk stratification remains an unsolved issue, hypermethylation of THOR was not only associated with higher *hTERT* expression but also with higher-risk disease in nonmuscle-invasive bladder cancers (pT1 high grade), and hypermethylation levels increased significantly in more severe cases (Leão R et al (2019) Combined genetic and epigenetic alterations of the TERT promoter affect clinical and biological behavior of bladder cancer. Int J Cancer 144(7):1676-1684).

Evidence for a prognostic value of increased THOR hypermethylation has also been provided for pancreatic cancer (Faleiro I et al (2017) The TERT hypermethylated oncologic region predicts recurrence and survival in pancreatic cancer. Future Oncol 13(23):2045-2051) and glioblastoma, where THOR methylation is a dynamic process during gliomagenesis (Castelo-Branco et al (2013) Methylation of the TERT promoter and risk stratification of childhood brain tumours: an integrative genomic and molecular study. Lancet Oncol. 14: 534-42), and for prostate cancer, where a possible risk stratification for low and intermediate Gleason cases by THOR hypermethylation has been suggested (Castelo-Branco et al (2016) A Cancer Specific Hypermethylation Signature of the TERT Promoter Predicts Biochemical Relapse in Prostate Cancer: A Retrospective Cohort Study. Oncotarget 7: 57726-57736). This is because THOR hypermethylation correlates with Gleason scores and has been found associated with tumor invasiveness (Qureshi SA, Bashir MU, Yaqinuddin A. (2010) Utility of DNA methylation markers for diagnosing cancer. Int J Surg. 8(3):194-8; Sidransky D. (2002) Emerging molecular markers of cancer. Nat Rev Cancer. 2(3):210-9).

While recent studies have shown a link between overall methylation in the THOR or the hTAPAS region and *hTERT* expression, and while *hTERT* expression has been linked to a number of cancers, there is still a need for improved methods of diagnosing, prognosing, and monitoring cancer, in particular those that are sensitive, specific, and cost-effective.

**SUMMARY OF THE INVENTION**

Against the aforementioned background, it is an object of the present invention to provide improved *in vitro* methods of diagnosing and monitoring tumors and/or prognosing tumors in histologically normal tissue. Another object is to provide improved *in vitro* methods that allow for the stratification of tumors by their grade, in particular of distinguishing high-grade tumors from low grade-tumors.

These objects are achieved by the provision of the primer of claim 1, the primer pair of claim 8, the *in vitro* assay kit of claim 9; the *in vitro* methods of diagnosing, prognosing, and/or monitoring a tumor according to claims 10 and 11; and the use according to claim 16.

In particular, the invention provides a methylation-specific primer for amplifying one or more CpG dinucleotides comprised in SEQ ID NO: 1.

The invention further provides a methylation-specific primer pair for amplifying one or more CpG dinucleotides comprised in the DNA region of SEQ ID NO: 1, wherein the primer pair comprises the methylation-specific primer of the invention and a primer that is methylation non-specific.

In addition, the invention provides an *in vitro* assay or *in vitro* assay kit comprising the methylation-specific primer of any one of claims 1 to 7, or the primer pair of claim 8.

In addition, the invention provides an *in vitro* method for diagnosing, prognosing, and /or monitoring a tumor, comprising

a) determining the level of methylation of one or more CpG dinucleotides comprised in one of SEQ ID NO: 1-9 of a test sample;
b) determining the level of methylation of the same one or more CpG dinucleotides comprised in one of SEQ ID NO: 1-9 of a control sample;
c) comparing the level of methylation of the one or more CpG dinucleotides of the test sample to that of the control sample;

wherein a hypermethylation in the test sample indicates the presence of a tumor, or indicates a high likelihood of developing a tumor; in particular of a urothelial bladder or prostate tumor.

The invention further provides an *in vitro* method for diagnosing, prognosing, and/or monitoring a tumor, comprising

(a) providing a test sample comprising or consisting of DNA;
(b) treating the DNA with sodium bisulfite to convert the unmethylated cytosines to uracil;
(c) amplifying the sodium bisulfite-converted DNA with one or more methylation-specific primers of any one of claims 1 to 7 or with one or more primer pairs of claim 8;
(d) determining the level of methylation of the amplified sodium bisulfite-converted DNA;
(e) repeating steps (a) to (d) for a control sample comprising or consisting of DNA;
(f) comparing the level of methylation of the amplified sodium bisulfite-converted DNA from the test sample to the level of methylation of the amplified sodium bisulfite-converted DNA from the control sample;

wherein a hypermethylation in the test sample as compared to the control sample indicates the presence of a tumor, or indicates a high likelihood of developing a tumor; in particular of a urothelial bladder or prostate tumor.

In addition, the invention provides a use of one or more of SEQ ID NO: 1-9, in particular of SEQ ID NO: 1 and/or 6 or a bisulfite-converted form thereof, in an *in vitro* method of diagnosing, prognosing, and/or monitoring cancer, in particular a bladder or prostate cancer.

By using the methylation-specific primers of the invention which are specific to the newly determined DNA region, the inventors provide a means to objectively and sensitively assay distinct new methylation patterns and by that diagnose, prognose, and monitor tumors, in particular urothelial tumors. In particular, the inventors found that a specific region upstream of the hTERT gene, namely a 1100-bp fragment (SEQ ID NO: 1), and in particular a 269-bp 5'-fragment (SEQ ID NO: 6) ranging from positions -658 to - 390 relative to the transcription start site AH007699.2 (i.e. of the *hTERT* gene), contains a high number of CpG dinucleotides that are differentially methylated in tumor specimens. By using the methylation-specific primers of the invention, which are specific to this newly determined DNA region, the inventors provide a means to objectively and sensitively diagnose, prognose, and monitor a tumor. In addition the inventors found that with their methylation-specific primers, even tissue samples adjacent to the tumor, which histologically were classified as non-tumor tissue, could be identified as already showing epigenetic alternations in their DNA methylation pattern of this specific region. Finally, the inventors by determining methylation patterns in particular of SEQ ID NO: 6 typical for low-grade and high-grade tumors provide a means of stratifying samples, further improving diagnostics. Furthermore, since the methods of the invention allow for detection of tumor stage specific methylation patterns from cell-free DNA of e.g. body fluids, the invention allows for early detection and monitoring of tumors.

Thus, the invention not only provides improved ways of detecting tumor-specific methylation patterns in biological samples, it also offers a sensitive, straightforward, and cost-efficient way of diagnosing and monitoring tumors *in vitro,* stratifying tumor tissue into low-grade and high-grade tumors, and even prognosing tumors in histologically-normal biological samples. Importantly, the methylation-specific primers and the methods comprising them are not limited to analyzing cellular DNA, but have the advantage that they can identify tumor-specific methylation patterns in cell-free DNA. Thus, the invention provides a robust way of diagnosing, pronosing, and/or monitoring tumors *in vitro.*

**DETAILED DESCRIPTION OF ILLUSTRATIVE EMBODIMENTS**

The inventors found that a specific 1100-bp 5'-fragment (SEQ ID NO: 1) ranging from positions -1051 to +49 relative to the transcription start site AH007699.2 contains CpG dinucleotides that are differentially methylated in tumors, in particular in urothelial tumors, and that a hypermethylation of the differentially methylated CpG dinucleotides in this region is also associated with a higher degree of *hTERT* methylation and a up-regulation of *hTERT* expression. The sequence of the sense strand of this DNA region is as follows (position -1051 as the first nucleotide, position +49 as the last AH007699.2 nucleotide; the transcriptional start site is italicized and underlined for reference purposes):

gggaagtcctcagctgtcctg**CG**gttgtgc**CG**gggccccaggtctggagggggaccagtggc**CG**tgtggcttctactgctggg

ctggaagt**CG**ggcctcctagctctgcagtc**CG**aggcttggagccaggtgcctggaccc**CG**aggctgccctccaccctgtg**CG**

gg**CG**ggatgtgaccagatgttggcctcatctgccagacagagtgc**CG**gggcccagggtcaaggc**CG**ttgtggctggtgtga

gg**CG**cc**CG**gtg**CGCG**gccagcaggag**CG**cctggctccatttcccaccctttct**CG**a**CG**ggac**CG**ccc**CG**gtgggtgatt

aacagatttggggtggtttgctcatggtggggacccct**CG**c**CG**cctgagaacctgcaaagagaaatga**CG**ggcctgtgtcaa

ggagcccaagt**CGCG**gggaagtgttgcagggaggcactc**CG**ggaggtcc**CGCG**tgcc**CG**tcagggagcaatg**CG**tcc

t**CG**ggtt**CG**tccccagc**CGCG**tcta**CGCG**cctc**CG**tcctccccttca**CG**tc**CG**gcatt**CG**tggtgcc**CG**gagcc**CG**a**C**

**G**ccc**CGCG**tc**CG**gacctggaggcagccctgggtctc**CG**gatcaggccag**CG**gccaaagggt**CG**c**CG**ca**CG**cacctgtt

cccagggcctccacatcatggcccctccct**CG**ggttaccccacagcctaggc**CG**att**CG**acctctctc**CG**ctggggccct**CG**

ctgg**CG**tccctgcaccctgggag**CGCG**ag**CG**g**CGCGCG**gg**CG**gggaag**CGCG**gcccagacccc**CG**ggtc**CG**gcc**C**

**G**gagcagctg**CG**ctgt**CG**gggccaggc**CG**ggctcccagtggatt**CGCG**ggcacaga**CG**cccaggac**CGCG**ctcccca

**CG**tgg**CG**gagggactggggacc**CG**ggcacc**CG**tcctgccccttaccttccagctc**CG**cctcctc**CGCGCG**gaccccgc

cc**CG**tcc**CG**acccctcc**CG**ggtccc**CG**gcccagcccccctc**CG**ggccctcccagcccctcccttcctttc**CGCG**gccc**CG**

ccctctcct**CGCG**g**CGCG**agtttcaggca*g***CG**ctg**CG**tcctgctg**CG**ca**CG**tgggaagccctggcccc

[SEQ ID NO: 1]

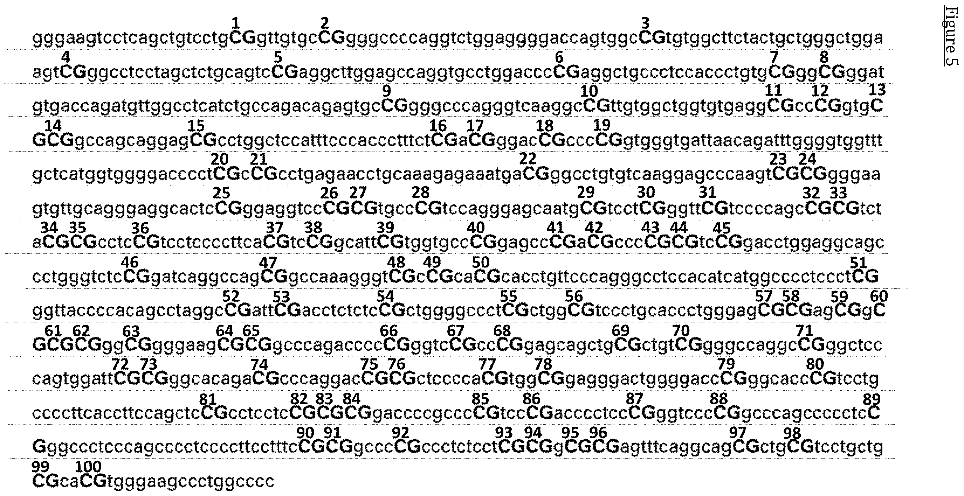

This DNA region contains 100 CpG dinucleotides, which are in capital letters and in bold for visualization purposes. The dinucleotides are numbered in Figure 5.

Following bisulfite conversion of SEQ ID NO:1 in a case where all of the CpG dinucleotides were methylated in SEQ ID NO:1, the converted, amplified DNA sequence is as follows (amplification occurs in the presence of thymine not uracil, and thus the converted uracils are replaced by thymines during amplification, resulting in the following sequence; the transcriptional start site is italicized and underlined for reference purposes):

gggaagtTTtTagTtgtTTtg**CG**gttgtg**TCG**ggg**TTT**Taggt**T**tggaggggaTTagtgg**TCG**tgtggTttTtaTt
gTtgggTtggaagt**CG**gg**TTt**TtagTt**T**tg**T**agt**TCG**aggTttggagTTaggtg**TT**tgga**TTT**CGaggTtg**TT**
TtTTaTTTtgtg**CG**gg**CG**ggatgtgaTTagatgttggTTtTatTtg**T**Taga**T**agagtg**TCG**ggg**TT**Tagggt**T**aa
gg**TCG**ttgtggTtggtgtgagg**CGTTCG**gtg**CGCG**gTTagTaggag**CGTT**ggTt**TT**atttTTTaTTTtttT**tC**
**G**a**CG**gga**TCGTTTCG**gtgggtgattaaTagatttggggtggtttgTtTatggtggggaTTTTt**CGTCG**TTtgagaa
TTtg**T**aaagagaaatga**CG**gg**TT**tgtgtTaaggagTTTaagt**CGCG**gggaagtgttgTagggaggTaTt**TCG**gga
ggtTT**CGCG**tgTT**CG**tTTagggagTaatg**CG**tTTt**CG**gttc**CG**tTTTTagt**CGCG**tTta**CGCG**TTt**TCG**tT
TtTTTTttTa**CG**t**TCG**gTatt**CG**tggtg**TT**CGgagTT**CGaCG**TTT**CGCG**t**TCG**gaTTtggaggTagTTTtg
ggtTt**TCG**gatTagg**TT**ag**CG**gTTaaaggg**TCGTCG**Ta**CG**Ta**TT**tgtt**TTT**Taggg**TT**tTTaTatTatggT**TT**
Tt**TTTt**CGgttaTTTTaTagTTtagg**TCG**att**CG**aTTtTtTt**CG**Ttgggg**TTT**t**CG**Ttgg**CG**tTTTtgTaT
TTtgggag**CGCG**ag**CG**g**CGCGCG**gg**CG**gggaag**CGCG**gTTTagaTTTT**CG**ggt**TCGTTCG**gagTagTtg
**CG**Ttgt**CG**ggg**TT**agg**TCG**gg**T**tTTTagtggatt**CGCG**ggTaTaga**CG**TTagga**TCGCG**TtTTTTa**CG**tg
g**CG**gaggga**T**tggggaTT**CG**gg**T**aTT**CG**tTTtgTTTTttTaTtttTTagTt**CG**Tt**TTt**T**CGCGCG**gaTT
TTg**TTTCG**tTT**CG**aTTTTt**TT**CGggtTTT**CG**gTTTagTTTTTt**CG**gg**TTT**tTTTagTTTTtTTTTtt
TTttt**TCGCG**gTTT**CGTTTt**Tt**TT**CGCGg**CGCG**agtttTagg**T**a*g***CG**Ttg**CG**tTTtgTtg**CG**Ta**CG**tggg
aagTTTtggTTTT

[SEQ ID NO: 2]

It will be evident to the skilled person that the uppercase "Ts" in the converted sequences can be understood to be etiher a "T" or a "U". Uracil is the product of bisulfite DNA treatment, but uracil is not added upon amplification of said DNA, and thus is only the template for the amplifications from the two original DNA strands. The terms "bisulfite" and "sodium bisulfite" are used interchangeably herein. The terms "bisulfite treatment", "bisulfite modification", "bisulfite conversion" are likewise used interchangeably herein and refer to the process described above where all cytosines in a DNA sample are converted to uracil upon sodium bisulfite treatment of DNA. The terms "bisulfite-converted DNA", "converted DNA", and "treated DNA" and the like are used interchangeably herein and refer to DNA that has undergone sodium bisulfite treatment to convert the cytosines to uracil.

The reverse and complement of the bisulfite converted sequence (i.e. of SEQ ID NO: 2) of a methylated allele is as follows:

AAAAccaAAActtcccaCGtACGcaAcaAAaCGcaACGCtAcctAaaactCGCGcCGCGaAAaAaAAACG
AAAcCGCGAaaaAAaaAAAAaAAAActAAAaAAAccCGAaAAAAActAAAcCGAAAaccCGAAaAA
AAtCGAAaCGAAAcAAAAtcCGCGCGAaAAaAACGAaActAAaaAAtAaaAAAAcaAAaCGAAtAcc
CGAAtccccaAtccctcCGccaCGtAAAAaACGCGAtcctAAACGtctAtAccCGCGaatccactAAAaAccC
GAcctAAcccCGacaACGcaActActcCGAACGAaccCGAAAAtctAAAcCGCGcttcccCGccCGCGCGc
CGctCGCGctcccaAAAtAcaAAAaCGccaACGaAAAccccaACGAaAaAaAAtCGaatCGAcctaAActA
tAAAAtaaccCGaAAAaAAAAccatAatAtAAaAAcctAAAacaAAtACGtACGACGaccctttAAcCGc
tAAcctAatcCGAaAacccaAAActAcctccaAAtcCGAaCGCGAAACGtCGAActcCGAAcaccaCGaatA
cCGAaCGtAaaAAAAaAAaCGAaAACGCGtaAaCGCGActAAAAaCGaaccCGaAAaCGcattActccc
tAAaCGAAcaCGCGAAacctccCGAaAtAcctccctAcaacacttcccCGCGacttAAActccttAacacaAAcc
CGtcatttctctttAcaAAttctcaAACGACGaAAAAtccccaccatAaAcaaaccacccccaaatctAttaatcacccac
CGAAACGAtccCGtCGaAaaaAAAtAAAaaatAAaAccaAACGctcctActAAcCGCGcacCGAACGcct
cacaccaAccacaaCGAccttAaccctAAAcccCGAcactctAtctAAcaAatAaAAccaacatctAAtcacatccC
GccCGcacaAAAtAAaAAAcaAcctCGAAAtccaAAcacctAActccaaAcctCGAactAcaAaActaAAaAA
ccCGacttccaAcccaAcaAtaAaaAccacaCGAccactAAtcccctccaAacctAAAAcccCGAcacaacCGcaA
AacaActAaAAacttccc

[SEQ ID NO: 3]

The bisulfite converted sequence of SEQ ID NO: 1, where all of the CpG dinucleotides were unmethylated is as follows:

gggaagtTTtTagTtgtTTtgTGgttgtgTTGgggTTTTaggtTtggagggggaTTagtggTTGtgtggTttTtaTt
gTtgggTtggaagtTGggTTtTTtagTtTtgTagtTTGaggTttggagTTaggtgTTtggaTTTTGaggTtgTT
TtTTaTTTtgtgTGggTGggatgtgaTTagatgttggTTtTatTtgTTagaTagagtgTTGgggTTTagggtTa

aggTTGttgtggTtggtgtgaggTGTTTGgtgTGTGgTTagTaggagTGTTtggTtTTatttTTTaTTTtttTt
TGaTGggaTTGTTTTGgtgggtgattaaTagatttggggtggtttgTtTatggtggggaTTTTtTGTTGTTtgag
aaTTtgTaaagagaaatgaTGggTTgtgtTaaggagTTTaagtTGTGgggaagtgttgTagggaggTaTtTTG
ggaggtTTTGTGtgTTTGtTTagggagTaatgTGtTTtTGggttTGtTTTTagTTGTGtTtaTGTGTTtT
TGtTTtTTTTtttTaTGtTTGgTattTGtggtgTTTGgagTTTGaTGTTTGTGtTTGgaTTggaggTag
TTTtgggtTtTTGgatTaggTTagTGgTTaaagggtTGTTGTaTGTaTTtgttTTTagggTTtTTaTatTat
ggTTTTtTTTtTGggttaTTTTaTagTTtaggTTGattTGaTTtTtTtTTGTtggggTTTtTGTtggTGtT
TTtgTaTTTtgggagTGTGagTGgTGTGTGGggTGgggaagTGTGgTTTagaTTTTTGggtTTGTTTG
gagTagTtgTGTtgtTGgggTTaggTTGggTtTTTagtggattTGTGgggTaTagaTGTTTaggaTTGTGT
tTTTTaTGtggTGgagggaTtggggaTTTGggTaTTTGtTTgTTTTttTaTTttTTagTtTTGTTtTTtT
TGTGTGgaTTTTgTTTTGtTTTGaTTTTtTTTGggtTTTTGgTTTagTTTTTtTTGggTTTtTTTa
gTTTTtTTTTttTTtttTTGTGgTTTTGTTTtTtTtTGTGgTGTGagtttTaggTa_gTGTtgTGtTTtg
TtgTGTaTGtgggaagTTTtggTTTT

[SEQ ID NO: 4]

The reverse and complement of the bisulfite converted sequence (i.e. of SEQ ID NO: 4) of an unmethylated allele is as follows:

AAAAccaAAActtcccaCAtACAcaAcaAAaCAcaACACtAcctAaaactCACAcCACAaAAaAaAAACA
AAAcCACAAaaaAAaaAAAAaAAAActAAAaAAAccCAAaAAAAActAAAcCAAAAaccCAAAaAA
AAtCAAAaCAAAAcAAAAtcCACACAAaAAaAACAAaActAAaaAAtAaaAAAAcaAAaCAAAtAcc
CAAAtccccaAtccctcCAccaCAtAAAAaACACAAtcctAAACAtctAtAccCACAaatccactAAAaAccC
AAcctAAcccCAacaACAcaActActcCAAACAAaccCAAAAAtctAAAcCACActtcccCAccCACACAcC
ActCACActcccaAAAtAcaAAAaCAccaACAaAAAccccaACAAaAaAaAAtCAaatCAAcctaAActAt
AAAAtaaccCAaAAAaAAAAccatAatAtAAaAAcctAAAaacaAAtACAtACAACAacccttAAcCAct
AAcctAatcCAAaAacccaAAActAcctccaAAtcCAAaCACAAAACAtCAAActcCAAAcaccaCAaatAc
CAAaCAtAaaAAAAaAAaCAAaAACACAtaAaCACAActAAAAaCAaaccCAaAAaCAcattActccctA
AaCAAAcaCACAAAacctccCAAaAtAcctccctAcaacacttcccCACAacttAAActccttAacacaAAccCAt
catttctctttAcaAAttctcaAACAACAaAAAAtccccaccatAaAcaaaccaccccaaatctAttaatcacccacCA
AAACAAtccCAtCAaAaaaAAAtAAAaaatAAaAccaAACActcctActAAcCACAcacCAAACAcctcaca
ccaAccacaaCAAccttAacccTAAAcccCAAcactctAtctAAcaAatAaAAccaacatctAAtcacatccCAccC
AcacaAAAtAAaAAAcaAcctCAAAAtccaAAcacctAActccaaAcctCAAactAcaAaActaAAaAAccCA

7

acttccaAcccaAcaAtaAaaAccacaCAAccactAAtcccctccaAacctAAAAcccCAAcacaacCAcaAAaca

ActAaAAacttccc

[SEQ ID NO: 5]

In addition, the inventors identified a specific 269-bp 5'-fragment of DNA within SEQ ID NO: 1, ranging from position -658 to -390 relative to the transcription start site *of hTERT* gene, that has a particularly high level of CpG dinucleotides that are differentially methylated in tumors, in particular in urothelial tumors, and that a hypermethylation of the differentially methylated CpG dinucleotides in this region is also associated with higher degree of *hTERT* methylation and up-regulation of *hTERT* expression, thus providing a particularly suitable DNA region to target in the context of the invention. The sequence of the sense strand of this DNA subregion is as follows (position -658 as the first nucleotide, position -390 as the last nucleotide):

Gggcctgtgtcaaggagcccaagt**CGCG**gggaagtgttgcagggaggcactc**CG**ggaggtcc**CGCG**tgcc**CG**tccagg

gagcaatg**CG**tcct**CG**ggtt**CG**tccccagc**CGCG**tcta**CGCG**cctc**CG**tcctcccccttca**CG**tc**CG**gcatt**CG**tggtgc

c**CG**gagcc**CG**a**CG**ccc**CGCG**tc**CG**gacctggaggcagccctgggtctc**CG**gatcaggccag**CG**gccaaagggt**CG**c

**CG**ca**CG**cacctgttcccagggcctccacatcatggc

[SEQ ID NO: 6]

Following bisulfite conversion of SEQ ID NO:6 in a case where all of the CpG dinucleotides were methylated in SEQ ID NO:6, the converted DNA sequence is as follows (here shown only with thymine, but uracil is the direct conversion nucleotide):

Ggg**TT**tgtgt**T**aaggag**TTT**aagt**CGCG**gggaagtgttg**T**aggggagg**T**a**T**t**T****CG**ggaggt**TT****CGCG**tg**TT****CG**t**T**

**T**agggag**T**aatg**CG**t**TT**t**CG**ggtt**CG**t**TTTT**ag**T****CGCG**t**T**ta**CGCG****TT**t**TCG**t**TT**t**TTTT**tt**T**a**CG**t**TCG**g

**T**att**CG**tggtg**TT****CG**gag**TT****CG**a**CG****TTT****CGCG**t**TCG**ga**TT**tggagg**T**ag**TTT**tgggt**T**t**T****CG**gat**T**agg**TT**

ag**CG**g**TT**aaaggggt**CG****TCG****T**a**CG****T**a**TT**tgtt**TTT**agggg**TT**t**TT**a**T**at**T**atgg**T**

[SEQ ID NO: 7]

The reverse and complement of the bisulfite converted sequence (i.e. of SEQ ID NO: 7) of a methylated allele is as follows:

Accat**A**at**A**t**AA**a**AA**ccct**AAA**aaca**AA**t**A****CG**t**A****CG**A**CG**acccttt**AA**c**CG**ct**AA**cct**A**atc**CG**A**A**a**A**ccca**A**

**AA**ct**A**cctcca**AA**tc**CG**Aa**CGCG****A****AA****CG**t**CG****AA**ctc**CG****AA**cacca**CG**aat**A**c**CG**Aa**CG**t**A**aa**AAAA**a**AA**a

**CG****A**a**AA****CGCG**ta**A**a**CGCG****A**ct**AAAA**a**CG**aacc**CG**a**AA**a**CG**catt**A**ctccct**AA**a**CG****AA**ca**CGCG****A****A**acct

cc**CG****A**a**A**t**A**cctccct**A**caacacttccc**CGCG**actt**AA****A**ctcctt**A**acaca**AA**cc**C**

[SEQ ID NO: 8]

Following bisulfite conversion of SEQ ID NO:6 in a case where all of the CpG dinucleotides were unmethylated in SEQ ID NO:6, the converted DNA sequence is as follows (here shown only with thymine, but uracil is the direct conversion nucleotide):

GggTTtgtgtTaaggagTTTaagt**TGTG**gggaagtgttgTagggaggTaTt**TTG**ggaggtTTT**GTG**tg**TTTG**t

TTagggagTaatg**TGt**TTt**TG**ggtt**TGt**TTTTag**TTGTG**tTta**TGTG**TTt**TTG**t**TTt**TTTTttTa**TGt**TT

Gg**Tatt**TGtggtgTTT**G**gagTTT**G**a**TG**TTT**TGTG**tTT**G**gaTTtggaggTagTTTtgggtTtTT**G**gatTagg

TTag**TG**g**TT**aaagggt**TGTTG**Ta**TG**TaTTtgttTTTagggTTtTTaTatTatggT

[SEQ ID NO: 9]

The reverse and complement of the bisulfite converted sequence (i.e. of SEQ ID NO: 9) of an unmethylated allele is as follows:

AccatAatAtAAaAAccctAAAaacaAAtACAtACAACAaccctttAAcCActAAcctAatcCAAaAacccaA

AActAcctccaAAtcCAAaCACAAAACAtCAAActcCAAAcaccaCAaatAcCAAaCAtAaaAAAAaAAa

CAAaAACACAtaAaCACAActAAAAaCAaaccCAaAAaCAcattActccctAAaCAAAcaCACAAAcctc

cCAAaAtAcctccctAcaacacttcccCACAacttAAActccttAacacaAAccC

[SEQ ID NO: 10]

Methylation-specific primer

In one aspect, the invention provides a methylation-specific primer for amplifying one or more CpG dinucleotides comprised in SEQ ID NO: 1. The primer of the invention is in particular specific to a part of SEQ ID NO: 1 that has been converted in a manner to distinguish methylated vs. unmethylated nucleotides, preferably methylated vs. unmethylated cytosines and in particular methylated vs. unmehtylated cytosines of one or more CpG dinucleotides. Typically the conversion is a bisulfite conversion or modification of the DNA (Herman et al. (1996) Methylation-specific PCR: A novel PCR assay for methylation status of CpG islands. PNAS USA, 93:9821-9826). In bisulfite treatment of DNA, cytosines are converted to uracil, but those that are methylated (5-methylcytosine) are resistant to this modification and remain as cytosine in the bisulfite-converted DNA (Wang, R. Y.-H., Gehrke, C. W. & Ehrlich, M. (1980) Nucleic Acids Res. 8, 4777-4790).

As such, it will be understood that while SEQ ID NO: 1 and 6 refer to the sequence of interest in the subject, when investigating the methylation pattern of said sequences, *in vitro* analysis is directed to the converted form of said sequences (e.g. SEQ ID NO: 2, 4, 7, and 9 or their reverse complements SEQ ID NO: 3, 5, 8, and 10, respectively). As such, when a primer is described to be specific to SEQ ID NO: 1 and/or 6, it is meant that it is specific to said sequences in their converted form, e.g. in their bisulfite converted form. This is especially the case in the context of bisulfite genomic sequencing and in all contexes involving the methylation-specific primers of the invention.

SEQ ID NO: 2 is the bisulfite-converted DNA sequence of SEQ ID NO: 1. It will be evident to the skilled person that the capitalized "T's" in SEQ ID NO: 2 represent the converted "U's", and that a primer that is specific for a converted "T" will be equally specific for a converted "U" at the same position. Thus, this has no effect on the primer. Experiments of the inventors found that methylation-specific primers that are specific for one or more CpG dinucleotides within SEQ ID NO: 1, in particular within the bisulfite-converted form of SEQ ID NO: 1 (i.e. SEQ ID NO: 2), provide a sensitive biomarker to differentiate healthy tissue from tumor tissue, in particular in bladder and/or prostate cancer.

In a preferred embodiment, the methylation-specific primer is specific to one or more CpG dinucleotides within SEQ ID NO: 6, in particular within the bisulfite-converted form of SEQ ID NO: 6 (i.e. SEQ ID NO: 7 or 9) or its reverse complement (i.e. SEQ ID NO: 8 or 10). In a particularly preferred embodiment, the methylation-specific primer of the invention comprises or consists of a sequence that is specific for two or more consecutive CpG dinucleotides, in particular for 2 or more, 3 or more, 4 or more, 5 or more, or 6 or more consecutive CpG dinucleotides within SEQ ID NO: 1 (e.g. SEQ ID NO: 2), and/or within SEQ ID NO: 6 (e.g. SEQ ID NO: 7), or their reverse complement sequence.

The term "specific" in the context of primers has its normal meaning in the art and refers to a sequence that can hybridize to a certain sequence of interest and preferably not to other sequences. A primer is considered to be specific herein when it has a sequence identity of 80% or more, 85% or more, 90% or more, 95% or more, 97% or more, 98% or more 99% or more, or 99.5% or more with the complement of a sequence of interest. Preferably, primers are considered specific herein when they have a sequence identity of 95% or more, or 97% or more with the complement of a sequence of interest. Most preferably, primers are considered specific herein when they have a 98% or more, 99% or more, or 99.5% or more sequence identity with the complement of a sequence of interest. In a particularly preferred embodiment, a primer is considered specific if it has a 100% sequence identity with the complement of a sequence of interest. In all cases, the primer is suitable for hybridizing to a sequence of interest and for priming DNA replication and/or amplification when hybridized.

The design of the primer follows the basic rules of primer design well known in the art, in particular for methylation-specific primers (Derks et al. (2004) Methylation-specific PCR unraveled. Cellular Oncology 26:291-299) and the skilled person is familiar with software tools available to support MS-PCR primer design (e.g. L.C. Li and R. Dahiya (2002) MethPrimer: designing primers for methylation PCRs, Bioinformatics 18:1427-1431; Rozen and H. Skaletsky (2000) Primer3 on the WWW for general users and for biologist programmers, Methods Mol. Biol. 132:365-386). For example, it is preferred that the primers are specific for sodium bisulfite converted DNA, preferably targeting a substantial number of non-CpG cytosines in the original pre-converted DNA. A substantial number in this context is for example 3 or more, preferably from 3 to 8. This reduces the chances of unconverted unmethylated CpG dinucleotide leading to an overrepresentation of methylation levels. It is also preferable that primers specific for one or more CpG dinucleotides are specific for one or more, two or more, or three or more CpG dinucleotides in the 3' region of the primers to increase the specificity of the primer annealing. Methylation-specific primers should also preferably end at a C within a CpG context, since in case of no methylation there is a mismatch at these final nucleotides and DNA polymerases do not synthesize when no double helix is available as a substrate.

Primers should also exclude pseudogenes and other closely related genomic sequences which could interfere with specific amplification. This can be accomplished, for example, by amplicon and primer sequences comparison in BLAT sequence database (https://genome.ucsc.edu/FAQ/FAQblat.html).

Furthermore, the primer should be suitable for amplification with PCR, in particular methylation-specific PCR. In a preferred embodiment, the primer of the invention has a melting temperature suitable for PCR, in particular from 45 °C to 65 °C, preferably from 55 °C to 65 °C. The skilled person is capable of determining appropriate lengths of primers based on, e.g. the desired sequence specificity and hybridization thermodynamics. Typically, primers are 18 or more bp in length to achieve gene specific primer annealing. In a preferred embodiment of the invention, the primers are 18 or more, 19 or more, 20 or more, 21 or more, 22 or more, 23 or more, 24 or more, 25 or more, or 26 or more bp in length. Typically, primers are 35 bp or less in length, preferably 30 bp or less in length. In a preferred embodiment, the primers are from 18 to 35 bp in length, more preferably from 18 to 30 bp in length or from 20 to 30 bp in length.

In some embodiments of the invention, the methylation-specific primer is specific for one or more CpG dinucleotides comprised in SEQ ID NO: 6 (or SEQ ID NO: 7), meaning one or more of CpG dinucleotide numbers 23 to 50 of SEQ ID NO. 1 (or SEQ ID NO: 2). In a preferred embodiment, the methylation-specific primer is specific for one or more CpG dinucleotides selected from the group consisting of CpG dinucleotides 23, 25, 26, 27, 31, 34, 35, 36, 39, 40, 43 and 47 of SEQ ID NO: 1 (or SEQ ID NO: 2). In a particularly preferred embodiment, the methylation-specific primer is specific for one or more CpG dinucleotides selected from the group consisting of CpG dinucleotides 25, 26, 27, 31, 34, 35, 36, 39, 40, 43 and 47 of SEQ ID NO: 1 (or SEQ ID NO: 2).

In certain preferred embodiments of the invention, the methylation-specific primer is specific for 2 or more consecutive CpG dinucleotides comprised in SEQ ID NO: 6 (or SEQ ID NO: 7), meaning two or more of CpG dinucleotide numbers 23 to 50 of SEQ ID NO: 1 (or SEQ ID NO: 2); preferably for 2, 3, 4, or 5 consecutive CpG dinucleotides. In a preferred embodiment, the CpG dinucleotides for which the methylation-specific primer is specific, comprise or consist of CpG dinucleotide number 23 and one of its two adjacent CpG dinucleotides, CpG dinucleotide 25 and one of its two adjacent CpG dinucleotides, CpG dinucleotide 26 and one of its two adjacent CpG dinucleotides, CpG dinucleotide 27 and one of its two adjacent CpG dinucleotides, CpG dinucleotide 31 and one of its two adjacent CpG dinucleotides, CpG dinucleotide 34 and one of its two adjacent CpG dinucleotides, CpG dinucleotide 35 and one of its two adjacent CpG dinucleotides, CpG dinucleotide 36 and one of its two adjacent CpG dinucleotides, CpG dinucleotide 39 and one of its two adjacent CpG dinucleotides, CpG dinucleotide 40 and one of its two adjacent CpG dinucleotides, CpG dinucleotide 43 and one of its two adjacent CpG dinucleotides, or CpG dinucleotide 47 and one of its two adjacent CpG dinucleotides.

The inventors also found that a number of CpG dinucleotides within SEQ ID NO: 1, and in particular within SEQ ID NO: 6, are particularly highly differentially methylated in high-grade tumor tissue and provide a particularly sensitive biomarker for the presence or prognosis of a high-grade tumor of the bladder and/or prostate. The CpG dinucleotides that were found to be particularly sensitive to high grade bladder cancer were CpG dinucleotides 28, 29, 30, 32, 33, 37, 38, 41, 42, 44, 45, and 46. The CpG dinucleotides that were identified to be particularly sensitive to high grade prostate cancer were CpG dinucleotides 24, 28, 29, 30, 32, 33, 37, 38, 41, 42, 44, 45, 46, 48, 49, and 50. In one embodiment, the methylation-specific primer is specific for one or more CpG dinucleotides selected from the group consisting of CpG dinucleotides 28, 29, 30, 32, 33, 37, 38, 41, 42, 44, 45, and 46; preferably selected from the group consisting of the CpG dinucleotides 28, 29, 32 and 33. In certain embodiments, the methylation-specific primer is specific for 2 or more, 3 or more, 4 or more, 5 or more, or 6 or more consecutive CpG dinucleotides within SEQ ID NO: 1 and/or 6, in particular their bisulfite-converted form (i.e. SEQ ID NO: 2 or 7, respectively), selected from the group consisting of CpG dinucleotides 28, 29, 30, 32, 33, 37, 38, 41, 42, 44, 45, and 46. Preferably, the methylation-specific primer is specific for 2, 3, 4, 5, or 6 consecutive CpG dinucleotides within SEQ ID NO: 1 and/or 6 (e.g. SEQ ID NO: 2 or 7), selected from the group consisting of CpG dinucleotides 28, 29, 30, 32, 33, 37, 38, 41, 42, 44, 45, and 46. In a particularly preferred embodiment, the methylation-specific primer is specific for 3, 4, or 5, preferably 4, consecutive CpG dinucleotides within SEQ ID NO: 1 and/or 6, selected from the group consisting of CpG dinucleotides 28, 29, 30, 32, 33, 37, 38, 41, 42, 44, 45, and 46.

In certain preferred embodiments of the invention, the methylation-specific primer is specific for 2 or more consecutive CpG dinucleotides, preferably for 2, 3, 4, or 5 consecutive CpG dinucleotides; wherein the CpG dinucleotides comprise or consist of CpG dinucleotide number 28 and one of its two adjacent CpG dinucleotides, CpG dinucleotide 29 and one of its two adjacent CpG dinucleotides, CpG dinucleotide 30 and one of its two adjacent CpG dinucleotides, CpG dinucleotide 32 and one of its two adjacent CpG dinucleotides, CpG dinucleotide 33 and one of its two adjacent CpG dinucleotides, CpG dinucleotide 37 and one of its two adjacent CpG dinucleotides, CpG dinucleotide 38 and one of its two adjacent CpG dinucleotides, CpG dinucleotide 41 and one of its two adjacent CpG dinucleotides, CpG dinucleotide 42 and one of its two adjacent CpG dinucleotides, CpG dinucleotide 44 and one of its two adjacent CpG dinucleotides, CpG dinucleotide 45 and one of its two adjacent CpG dinucleotides, or CpG dinucleotide 46 and one of its two adjacent CpG dinucleotides.

The term "adjacent" as used herein in the context of CpG dinucleotides refers to the next consecutive CpG dinucleotide in either direction. Each of the CpG dinucleotides of SEQ ID NO: 1 and/or 6, except the first and the last one, has two adjacent CpG dinucleotides within the sequence - one located upstream and one located downstream. In some cases, the CpG dinucleotides are numerically and spatially adjacent to each other; in other cases, two adjacent CpG dinucleotides have one or more non-CpG nucleotides between them.

In one embodiment of the invention, the primer of the invention is specific for 3 or more, 4 or more, or 5 or more consecutive CpG dinucleotides, preferably for 3, 4, or 5 consecutive CpG dinucleotides; wherein the CpG dinucleotides comprise or consist of CpG dinucleotide number 28 and its two adjacent CpG dinucleotides, CpG dinucleotide 29 and its two adjacent CpG dinucleotides, CpG dinucleotide 30 and its two adjacent CpG dinucleotides, CpG dinucleotide 32 and its two adjacent CpG dinucleotides, CpG dinucleotide 33 and its two adjacent CpG dinucleotides, CpG dinucleotide 37 and its two adjacent CpG dinucleotides, CpG dinucleotide 38 and its two adjacent CpG dinucleotides, CpG dinucleotide 41 and its two adjacent CpG dinucleotides, CpG dinucleotide 42 and its two adjacent CpG dinucleotides, CpG dinucleotide 44 and its two adjacent CpG dinucleotides, CpG dinucleotide 45 and its two adjacent CpG dinucleotides, or CpG dinucleotide 46 and its two adjacent CpG dinucleotides. In a particularly preferred embodiment, the primer of the invention is specific for 3 or more, 4 or more, or 5 or more consecutive CpG dinucleotides, preferably for 3, 4, or 5 consecutive CpG dinucleotides; wherein the CpG dinucleotides comprise or consist of CpG dinucleotide number 28 and its two adjacent CpG dinucleotides, CpG dinucleotide 29 and its two adjacent CpG dinucleotides, or CpG dinucleotide 30 and its two adjacent CpG dinucleotides.

The numbering of the 100 CpG dinucleotides within the DNA region SEQ ID NO: 1 is as follows: CpG dinucleotide 1 refers to the farthest "upstream" position relative to the hTERT transcription start site in the sense strand; CpG dinucleotide 100 refers to the CpG dinucleotide farthest "downstream", relative to the hTERT transcription start site in the sense strand. The specific location of the cytosine of each CpG dinucleotide in the DNA region SEQ ID NO:1 is listed in Table 1 below.

**Table 1. CpG dinucleotide numbering within DNA region SEQ ID NO: 1**

| CpG No. | DNA position relative to hTERT transcription start site | CpG No. | DNA position relative to hTERT transcription start site | CpG No. | DNA position relative to hTERT transcriptio n start site |
|---|---|---|---|---|---|
| 1 | -1030 | 35 | -540 | 69 | -245 |
| 2 | -1021 | 36 | -534 | 70 | -239 |
| 3 | -990 | 37 | -520 | 71 | -228 |
| 4 | -961 | 38 | -516 | 72 | -211 |
| 5 | -939 | 39 | -509 | 73 | -209 |
| 6 | -911 | 40 | -500 | 74 | -199 |
| 7 | -889 | 41 | -493 | 75 | -189 |
| 8 | -885 | 42 | -490 | 76 | -187 |
| 9 | -842 | 43 | -485 | 77 | -178 |
| 10 | -823 | 44 | -483 | 78 | -173 |

(continued)

| CpG No. | DNA position relative to hTERT transcription start site | CpG No. | DNA position relative to hTERT transcription start site | CpG No. | DNA position relative to hTERT transcriptio n start site |
|---|---|---|---|---|---|
| 11 | -804 | 45 | -479 | 79 | -156 |
| 12 | -800 | 46 | -453 | 80 | -148 |
| 13 | -795 | 47 | -440 | 81 | -122 |
| 14 | -793 | 48 | -428 | 82 | -113 |
| 15 | -780 | 49 | -425 | 83 | -111 |
| 16 | -753 | 50 | -421 | 84 | -109 |
| 17 | -750 | 51 | -381 | 85 | -97 |
| 18 | -744 | 52 | -360 | 86 | -92 |
| 19 | -739 | 53 | -354 | 87 | -82 |
| 20 | -689 | 54 | -344 | 88 | -74 |
| 21 | -686 | 55 | -332 | 89 | -59 |
| 22 | -659 | 56 | -326 | 90 | -29 |
| 23 | -634 | 57 | -307 | 91 | -27 |
| 24 | -632 | 58 | -305 | 92 | -21 |
| 25 | -606 | 59 | -300 | 93 | -10 |
| 26 | -596 | 60 | -297 | 94 | -8 |
| 27 | -594 | 61 | -295 | 95 | -5 |
| 28 | -588 | 62 | -293 | 96 | -3 |
| 29 | -572 | 63 | -289 | 97 | +12 |
| 30 | -566 | 64 | -281 | 98 | +17 |
| 31 | -560 | 65 | -279 | 99 | +27 |
| 32 | -550 | 66 | -266 | 100 | +31 |
| 33 | -548 | 67 | -260 | | |
| 34 | -542 | 68 | -256 | | |

The methylation-specific primer of the invention can be specific to the methylated form or the unmethylated form of one or more CpG dinucleotides. In a preferred embodiment, the methylation-specific primer of the invention is specific to the fully methylated form of one or more CpG dinucleotides, in particular to all of the CpG dinucelotides to which it is specific (e.g. SEQ ID NO: 2 or 7, or their reverse complements). In another preferred embodiment, the methylation-specific primer of the invention is specific to the fully unmethylated form of one or more CpG dinucleotides, which following conversion are TpG dinucleotides (e.g. SEQ ID NO: 4 or 9, or their reverse complements). In some embodiments, the methylation-specific primer of the invention is specific to a mixure of methylated and unmethylated forms of different CpG dinucleotides. Examples of exemplary DNA sequences of methylation-specific primers of the invention are listed in Tables 2 and 3.

**Table 2. Sequences of exemplary MS-PCR primers (forward sense)**

| SEQ ID NO: | DNA sequence | SEQ ID NO: | DNA sequence |
|---|---|---|---|
| 11 | tTTtTagTtgtTTtg**CG**gttgtgT**C** | 51 | Tt**CG**ggttaTTTTaTagTTtagg**TC** |
| 12 | tt**CG**aggTtgTTTtTTaTTTtgtg**C** | 52 | gttaTTTTaTagTTtagg**TCG**att**C** |
| 13 | aggTtgTTTtTTaTTTtgtg**CG**gg**C** | 53 | gtttagg**TCG**att**CG**aTTtTtTt**C** |

(continued)

| SEQ ID NO: | DNA sequence | SEQ ID NO: | DNA sequence |
|---|---|---|---|
| 14 | agtgT**CG**gggTTTagggtTaaggT**C** | 54 | t**CG**aTTtTtTtT**CG**TtggggTTTt**C** |
| 15 | aaggT**CG**ttgtggTtggtgtgagg**C** | 55 | tTtTtT**CG**TtggggTTTt**CG**Ttgg**C** |
| 16 | tc**G**ttgtggTtggtgtgagg**CGTTC** | 56 | **G**Ttgg**CG**tTTTTtgTaTTTtgggag**C** |
| 17 | gtggTtggtgtgagg**CGTTCG**gtg**C** | 57 | tgg**CG**tTTTTtgTaTTTtgggag**CGC** |
| 18 | ggTtggtgtgagg**CGTTCG**gtg**CGC** | 58 | **G**tTTTTtgTaTTTtgggag**CGCG**ag**C** |
| 19 | cgtT**CG**gtg**CGCG**gTTagTaggag**C** | 59 | tttgTaTTTtgggag**CGCG**ag**CG**g**C** |
| 20 | TtggTtTTatttTTTaTTTttttTt**C** | 60 | tgTaTTTtgggag**CGCG**ag**CG**g**C**gc |
| 21 | gttTTatttTTTaTTTttttTt**CG**a**C** | 61 | TaTTTtgggag**CGCG**ag**CG**g**CGCGC** |
| 22 | tttTTTaTTTttttTt**CG**a**CG**gga**TC** | 62 | Ttgggag**CGCG**ag**CG**g**CGCGCG**gg**C** |
| 23 | TaTTTttttTt**CG**a**CG**gga**TCG**TTT**C** | 63 | **GCG**ag**CG**g**CGCGCG**gg**CG**gggaag**C** |
| 24 | ttgTtTatggtggggaTTTTt**CG**T**C** | 64 | **G**ag**CG**g**CGCGCG**gg**CG**gggaag**CGC** |
| 25 | **G**ggTTgtgtTaaggagTTTaagt**C** | 65 | g**CG**gggaag**CGCG**gTTagaTTTT**C** |
| 26 | gTTgtgtTaaggagTTTaagt**CGC** | 66 | aag**CGCG**gTTagaTTTT**CG**ggt**TC** |
| 27 | gTagggaggTaTt**CG**ggaggtTT**C** | 67 | **GCG**gTTagaTTTT**CG**ggt**TCG**TT**C** |
| 28 | agggaggTaTt**CG**ggaggtTT**CGC** | 68 | TTT **G**ggt**TCG**TT**CG**gagTagTtg**C** |
| 29 | gtattT**CG**ggaggtTT**CGCG**tgTT**C** | 69 | gt**CG**TT**CG**gagTagTtg**CG**Ttgt**C** |
| 30 | cgc**G**tgTT**CG**tTTagggagTaatg**C** | 70 | gTagTtg**CG**Ttgt**CG**gggTTagg**TC** |
| 31 | TT**CG**tTTagggagTaatg**CG**tTTt**C** | 71 | gTTagg**TCG**ggTtTTTagtggatt**C** |
| 32 | tagggagTaatg**CG**tTTt**CG**ggtt**C** | 72 | Tagg**TCG**ggTtTTTagtggatt**CGC** |
| 33 | tg**CG**tTTt**CG**ggtt**CG**tTTTTTagT**C** | 73 | ttttagtggatt**CGCG**ggTaTaga**C** |
| 34 | c**G**tTTt**CG**ggtt**CG**tTTTTTagT**CGC** | 74 | ttcg **CG**ggTaTaga**CG**TTagga**TC** |
| 35 | c**G**ggtt**CG**tTTTTTagT**CGCG**tTta**C** | 75 | cg**CG**ggTaTaga**CG**TTagga**TCGC** |
| 36 | ggtt**CG**tTTTTTagT**CGCG**tTta**CGC** | 76 | aga**CG**TTagga**TCGCG**TtTTTTTa**C** |
| 37 | tttTTagT**CGCG**tTta**CGCG**TTtT**C** | 77 | TTTagga**TCGCG**TtTTTTTa**CG**tgg**C** |
| 38 | tac**GCG**TTtT**CG**tTTtTTTTTttTa**C** | 78 | t a**CG**tgg**CG**gaggggaTtggggaTT**C** |
| 39 | c**G**TTtT**CG**tTTtTTTTTttTa**CG**tT**C** | 79 | **G**gaggggaTtggggaTT**CG**ggTaTT**C** |
| 40 | **G**tTTtTTTTTttTa**CG**tT**CG**gTatt**C** | 80 | ttTaTTttTTagTtt**CG**TTtTTttT**C** |
| 41 | ttTa**CG**tT**CG**gTatt**CG**tggtgTT**C** | 81 | t aTTttTTagTtt**CG**TTtTTttT**CGC** |
| 42 | T**CG**gTatt**CG**tggtgTT**CG**gagTT**C** | 82 | ttt **tTTagTtTCGTTtTTtTCGCGC** |
| 43 | gTatt**CG**tggtgTT**CG**gagTT**CG**a**C** | 83 | **G**TTtTTtT**CGCGCG**gaTTTTTgTTT**C** |
| 44 | **CG**tggtgTT**CG**gagTT**CG**a**CG**TTT**C** | 84 | TtT**CGCGCG**gaTTTTTgTTT**CG**tTT**C** |
| 45 | tggtgTT**CG**gagTT**CG**a**CG**TTT**CGC** | 85 | aTTTTTgTTT**CG**tTT**CG**aTTTTtTT**C** |
| 46 | gTT**CG**gagTT**CG**a**CG**TTT**CGCG**tT**C** | 86 | t **CG**tTT**CG**aTTTTtTT**CG**ggtTTT**C** |
| 47 | tTTtgggtTtT**CG**gatTaggTTag**C** | 87 | **TCG**ggtTTT**CG**gTTTagTTTTTTtT**C** |
| 48 | ggatTaggTTag**CG**gTTaaagggt**C** | 88 | tttttt TTttTTttttT**CGCG**gTTT**C** |
| 49 | tTaggTTag**CG**gTTaaagggt**CGTC** | 89 | tttt**TCGCG**gTTT**CG**TTTtTtTTt**C** |
| 50 | gTTag**CG**gTTaaagggt**CGTCG**Ta**C** | 90 | tt**TCGCG**gTTT**CG**TTTtTtTTt**CGC** |

**Table 3. Sequences of exemplary MS-PCR primers (reverse antisense)**

| SEQ ID NO: | DNA sequence | SEQ ID NO: | DNA sequence |
|---|---|---|---|
| 91 | AAActtcccaCGtACGcaAcaAAaC | 133 | caAAAaCGccaACGaAAAccccaAC |
| 92 | tcccaCGtACGcaAcaAAaCGcaAC | 134 | ACGaAAAccccaACGAaAaAaAAtC |
| 93 | caAAaCGcaACGCtAcctAaaactC | 135 | AAccccaACGAaAaAaAAtCGaatC |
| 94 | AAaCGcaACGCtAcctAaaactCGC | 136 | atCGAcctaAActAtAAAAtaaccC |
| 95 | CGcaACGCtAcctAaaactCGCGcC | 137 | AAaAAccctAAAaacaAAtCGtAC |
| 96 | caACGCtAcctAaaactCGCGcCGC | 138 | aaccctAAAaacaAAtCGtACGAC |
| 97 | AaaactCGCGcCGCGaAAaAaAAAC | 139 | aAAtCGtACGACGaccctttAAcC |
| 98 | cGCGcCGCGaAAaAaAAACGAAAcC | 140 | gaccctttAAcCGctAAcctAatcC |
| 99 | CGcCGCGaAAaAaAAACGAAAcCGC | 141 | cccaAAActAcctccaAAtcCGAaC |
| 100 | AAAaAAAccCGaAAAAAActAAAcC | 142 | caAAActAcctccaAAtcCGAaCGC |
| 101 | cCGaAAAAAActAAAcCGAAAaccC | 143 | ctAcctccaAAtcCGAaCGCGAAAC |
| 102 | ctaAAcCGAAAaccCGAAaAAAAtC | 144 | cctccaAAtcCGAaCGCGAAACGtC |
| 103 | cCGAAAaccCGAAaAAAAtCGAAaC | 145 | AtcCGAaCGCGAAACGtCGAActcC |
| 104 | aaAAAAtCGAAaCGAAAcAAAAtcC | 146 | cGAAACGtCGAActcCGAAcaccaC |
| 105 | aaaatCGAAaCGAAAcAAAAtcCGC | 147 | tCGAActcCGAAcaccaCGaatAcC |
| 106 | AAtCGAAaCGAAAcAAAAtcCGCGC | 148 | ActcCGAAcaccaCGaatAcCGAaC |
| 107 | GAAAcAAAAtcCGCGCGAaAAaAAC | 149 | GaatAcCGAaCGtAaaAAAAaAAaC |
| 108 | aAAtAaaAAAAcaAAaCGAAtAccC | 150 | cGAaCGtAaaAAAAaAAaCGAaAAC |
| 109 | GAAtAccCGAAtccccaAtccctcC | 151 | AaCGtAaaAAAAaAAaCGAaAACGC |
| 110 | ccCGAAtccccaAtccctcCGccaC | 152 | aaAAAAaAAaCGAaAACGCGtaAaC |
| 111 | ccaAtccctcCGccaCGtAAAAaAC | 153 | AAAAaAAaCGAaAACGCGtaAaCGC |
| 112 | aAtccctcCGccaCGtAAAAaACGC | 154 | AaAACGCGtaAaCGCGActAAAAaC |
| 113 | ccaCGtAAAAaACGCGAtcctAAAC | 155 | CGtaAaCGCGActAAAAaCGaaccC |
| 114 | aACGCGAtcctAAACGtctAtAccC | 156 | CGCGActAAAAaCGaaccCGaAAaC |
| 115 | CGCGAtcctAAACGtctAtAccCGC | 157 | ccCGaAAaCGcattActccctAAaC |
| 116 | AtAccCGCGaatccactAAAaAccC | 158 | AaCGcattActccctAAaCGAAcaC |
| 117 | tccactAAAaAccCGAcctAAcccC | 159 | CGcattActccctAAaCGAAcaCGC |
| 118 | AAAaAccCGAcctAAcccCGacaAC | 160 | cctAAaCGAAcaCGCGAAacctccC |
| 119 | ctAAcccCGacaACGcaActActcC | 161 | AtAcctccctAcaacacttcccCGC |
| 120 | cccCGacaACGcaActActcCGAAC | 162 | GacttAAActccttAacacaAAccC |
| 121 | caACGcaActActcCGAACGAaccC | 163 | ttctctttAcaAAttctcaAACGAC |
| 122 | cCGAACGAaccCGAAAAtctAAAcC | 164 | aaatctAttaatcacccacCGAAAC |
| 123 | GAACGAaccCGAAAAtctAAAcCGC | 165 | AttaatcacccacCGAAACGAtccC |
| 124 | cCGAAAAtctAAAcCGCGcttcccC | 166 | aatcacccacCGAAACGAtccCGtC |
| 125 | AAAtctAAAcCGCGcttcccCGccC | 167 | atAAaAccaAACGctcctActAAcC |
| 126 | atctAAAcCGCGcttcccCGccCGC | 168 | aaaAccaAACGctcctActAAcCGC |
| 127 | ctAAAcCGCGcttcccCGccCGCGC | 169 | caAACGctcctActAAcCGCGcacC |

(continued)

| SEQ ID NO: | DNA sequence | SEQ ID NO: | DNA sequence |
|---|---|---|---|
| 128 | AAc**CGCG**cttccc**CG**cc**CGCGCG**c**C** | 170 | cgctcctActAAc**CGCG**cac**CG**AA**C** |
| 129 | g**CG**cttccc**CG**cc**CGCGCG**c**CG**ct**C** | 171 | c**CG**AA**CG**cctcacaccaAccacaa**C** |
| 130 | **G**cttccc**CG**cc**CGCGCG**c**CG**ct**CGC** | 172 | cacaa**CG**Acctt**A**accct**A**AAcc**C** |
| 131 | gct**CGCG**ctcccaAAAtAcaAAAa**C** | 173 | AccaacatctAAtcacatcc**CG**cc**C** |
| 132 | **G**ctcccaAAAtAcaAAAa**CG**cca**C** | 174 | **cccG**cacaAAAtAAaAAAcaAcct**C** |

In one embodiment, the methylation-specific primer of the invention comprises or consists of a nucleic acid sequence that has 80% or more sequence identity to one of SEQ ID NO: 11-90 (forward sense primer), preferably to one of SEQ ID NO: 25-50. In another embodiment, the methylation-specific primer of the invention comprises or consists of a nucleic acid sequence that has 80% or more sequence identity to one of SEQ ID NO: 91-174 (reverse antisense primer), preferably to one of SEQ ID NO: 137-161.

In one embodiment, the methylation-specific primer of the invention comprises or consists of a nucleic acid sequence that has 90% or more, 95% or more; preferably 98% or more, 99% or more, or 99.5% or more; most preferably a 100% sequence identity, to one of SEQ ID NO: 11-90 (forward sense primer), preferably to one of SEQ ID NO: 25-50. In another embodiment, the methylation-specific primer of the invention comprises or consists of a nucleic acid sequence that has 90% or more, 95% or more; preferably 98% or more, 99% or more, or 99.5% or more; most preferably a 100% sequence identity, to one of SEQ ID NO: 91-174 (reverse antisense primer), preferably to SEQ ID NO: 137-161.

In a preferred embodiment, the nucleotides of the methylation-specific primer for the sense strand of DNA are completely complementary to 90% or more, 95% or more; preferably 98% or more, 99% or more, or 99.5% or more; most preferably to 100% of the nucleotides in one of SEQ ID NO: 25-50 for the sense strand, and the nucleotides of the methylation-specific primer for the antisense strand of DNA are completely complementary to 90% or more, 95% or more; preferably 98% or more, 99% or more, or 99.5% or more; most preferably to 100% of the nucleotides in SEQ ID NO: 91-174 (reverse antisense primer), preferably to SEQ ID NO: 137-161.

Methylation-specific primer pair

In one aspect of the invention, a methylation-specific primer pair for amplifying one or more CpG dinucleotides comprised in the DNA region of SEQ ID NO: 1, in particular within SEQ ID NO: 1 following bisulfite conversion, is provided.

A "primer pair" as defined herein refers to two primers, one of which is the methylation-specific primer of the invention, in particular a primer that is specific for the methylated state of one or more CpG dinucleotides of interest in the DNA region of SEQ ID NO:1 and/or 6, and the other primer in the primer pair is specific for an unmethlyated sequence, in order to take advantage of the sequence differences resulting from bisulfite modification of the DNA (Herman et al. (1996) Methylation-specific PCR: A novel PCR assay for methylation status of CpG islands. PNAS USA, 93:9821-9826). An MS-PCR primer specific for an unmethylated DNA sequence (i.e. the unmethylation-specific primer) will only amplify sodium bisulfite converted DNA in which the cytosines of interest have been converted to uracil at this position. Likewise, the primer specific for a methylated DNA sequence (i.e. the methylation-specific primer) will only amplify sodium bisulfite converted DNA in which the cytosines are still present in the positions of interest, e.g. in differentially methylated CpG dinucleotides. The primers of the primer pair should be specific to sequences in the same strand. In a preferred embodiment, the primer of the primer pair specific for the unmethylated sequence is specific for a sequence without CpGs. This ensures that this primer does not have preference for methylation pattern, i.e. is methylation non-specific.

In certain embodiments, the methylation-specific and methylation-non-specific primers of the primer pair are specific for the same or different DNA regions. In both cases, the methylation-specific primer is specific to a DNA sequence comprising one or more CpG dinucleotides of interest. In a preferred embodiment, the primer pair consists of a methylation-specific primer and a methylation-non-specific primer, wherein the methylation-non-specific primer is specific to a DNA sequence comprising no CpG dinucleotides, and preferably is close to or adjacent to the DNA sequence targeted by the methylation-specific primer of the primer pair.

The primers of the primer pair may be of the same or different lengths. It is preferable to design both primers of the primer pair with similar melting temperatures, preferably from 55 °C to 65 °C. A similar melting temperature as defined herein is a melting temperature that differs from another melting temperature by 3 °C or less, preferably by 2 °C or less, or by 1 °C or less. In one embodiment, the primers of the primer pair have a melting temperature that differs by 3 °C or less, preferably by 2 °C or less or 1 °C or less. Most preferably, the primers of the primer pair have a melting temperature that differs from one another by 1 °C or less, 0.5 °C or less, 0.2 °C or less, or 0.1 °C or less. Since the sodium bisulfite conversion decreases the GC content of the unmethylation-specific primer, typically the sequence of the unmethylation-specific primer is longer than the methylation-specific primer. In particular, it may be extended into the 5' region. Thus, in one embodiment, the primers of the primer pair are of the different lengths, in particular, the unmethylation-specific primer is longer than the methylation-specific primer. This allows paired analyses in a single thermocycler and provides a convenient way to recognize each amplicon after gel electrophoresis.

It will be evident that for every "forward sense" primer, there should be a corresponding "reverse antisense" primer. This ensures exponential amplification of a sequence of interest. The primers of a primer pair amplify a sequence on the same strand. Thus, by a pair of primers, it is understood that this refers to a pair of primer pairs, e.g. to: methylation-specific primer (forward sense and reverse antisense) and methylation-non-specific primer (forward sense and reverse antisense).

In certain embodiments, the DNA region without CpG dinucleotides is idiolocal to SEQ ID NO: 1 and/or 6 (or their bisulfite converted forms), preferably idiolocal to the sequence amplified by the methylation-specific primer aspect of the invention. This embodiment is particularly useful when there is known or expected genomic variation in the samples to be tested, as it allows for accurate measurement of DNA methylation differences in samples with varying numbers of target loci due to e.g. genome instability. These primer pairs are particularly suitable to be used in an idiolocal normalized real-time methylation-specific PCR method (Santourlidis et al. (2016) IDLN-MSP: Idiolocal normalization of real-time methylation-specific PCR for genetic imbalanced DNA specimens. Biotechniques. 60(2):84-7). In these embodiments, the primers of the primer pair are preferably specific for DNA regions that are relatively close to one another, for example located on the same chromosome, e.g. on chromosome 5. Chromosome 5 has about 181 million basepairs. In some embodiments, the two idiolocal DNA regions have 181 million bp or less, 100 million bp or less, 50 million bp or less, 25 million bp or less, or preferably 1 million bp or less between each other. In some embodiments, the two idiolocal DNA regions have 6000 bp or less, 5000 bp or less, 4000 bp or less, 3000 bp or less, 2000 bp or less, 1000 bp or less, 900 bp or less, 800 bp or less, 700 bp or less, 600 bp or less in between each other, preferably 500 bp or less, 400 bp or less, 300 bp or less, 200 bp or less, or 100 bp or less, between each other. More preferably, there are 100 bp or less, 75 bp or less, 50 bp or less, 25 bp or less, or 10 bp or less separating the two DNA regions. In some embodiments, the two idiolocal DNA regions are adjacent to one another, meaning they are separated by 10 bp or less, or more preferably 0 bp. In some embodiments, the two primers of the primer pair are specific for overlapping sequences. In selecting the two idiolocal DNA regions to be close to one another, the copy numbers of both sequences will be identical in most cases, resulting in accurate normalization and reliable comparative measurements of DNA methylation when using real-time MS-PCR, even for clinical samples.

It will be apparent to the skilled person that the invention is not limited to a single primer or a single primer pair, but extends to a set of the methylation-specific primers of the invention, and to a set of primer pairs according to the invention.

Set of the methylation-specific primers or the primer pairs of the invention

In another aspect, the invention provides a set of methylation-specific primers or a set of primer pairs. The two or more different primers or primer pairs are specific for different methylation patterns of one or more CpG dinucleotides comprised or consisted in SEQ ID NO: 1 and/or 6.

In one embodiment, the set comprises or consists of 2 to 50 different methylation-specific primers or primer pairs of the invention, preferably wherein each methylation-specific primer is specific for 1, 2, 3, 4, 5, or more different CpG dinucleotides within SEQ ID NO: 1, preferably within SEQ ID NO: 6. Preferably, the set according to the invention comprises from 2 to 25, from 2 to 20, from 2 to 15, from 2 to 10, or from 2 to 5 different methylation-specific primers, wherein each primer is preferably specific for 1, 2, 3, 4, 5, or more different CpG dinucleotides within SEQ ID NO: 1, preferably within SEQ ID NO: 6. The different methylation-specific primers differ in their specificity for one or more CpG dinculeotides, in particular for one or more CpG dinucleotides of SEQ ID NO:1, preferably of SEQ ID NO: 6. The different methylation-specific primers of the set may have overlapping specificity to one or more CpG dinucleotides. In one embodiment, each methylation-specific primer of the set is specific for 1 CpG dinucleotide. In another embodiment, each methylation-specific primer of the set is specific for 2, 3, 4, 5 or more, preferably 2, 3, 4, or 5 CpG dinucleotides in SEQ ID NO:1, preferably in SEQ ID NO: 6.

Use in a MS-PCR method

In another aspect, the invention provides the use of the methylation-specific primer, the primer pair, or the set according to the previous aspects of the invention, to prime *in vitro* DNA synthesis, in particular in a methylation-specific PCR method.

The methylation-specific primer and primer pair, as well as the set according to the invention are all suitable for use in a methylation-specific PCR method, in particular to prime *in vitro* DNA synthesis in a methylation-specific PCR method and thus amplify a target nucleic acid sequence, in particular a target DNA sequence in sodium bisulfite-converted DNA. Thus, in a further aspect, the invention provides the use of the methylation-specific primer, primer pair, and/or set of the invention to amplify one or more CpG dinucleotides comprised in SEQ ID NO: 1 and/or 6, in particular their sodium bisulfite-converted form, e.g. SEQ ID NO: 2, 3, 4, 5, 7, 8, 9, or 10, preferably SEQ ID NO: 7, 8, 9, or 10.

By using the PCR step to distinguish methylated from unmethylated DNA, MS-PCR bypasses the more time-consuming and expensive methods of genomic sequencing, offering a high level of high level of sensitivity (Herman et al. (1996) Methylation-specific PCR: A novel PCR assay for methylation status of CpG islands. PNAS USA, 93:9821-9826). Methylation-specific PCR methods are known to the skilled person. In one embodiment, the methylation-specific PCR method comprises a singleplex MS-PCR method, a mutiplex MS-PCR method, a quantitative MS-PCR method, an in situ MS-PCR method, an HPLC method, or a DGGE method. In certain embodiments, the methylation-specific PCR method is a semi-quantitative method, such as intercalating dye real-time MS-PCR. In a particularly preferable embodiment, the methylation-specific PCR method is a quantitative MS-PCR method, preferably a real-time MS-PCR method. In a particularly preferable embodiment, the methylation-specific primer, primer pair, or set of the invention is used in an idiolocal normalized real-time methylation specific PCR method.

In vitro assay

In another aspect, the invention provides an *in vitro* assay or *in vitro* assay kit comprising the methylation-specific primer, primer pair, or set provided herein. The assay and kit is for performing PCR, in particular methylation-specific PCR. The kits may further comprise other components suitable for performing methylation-specific PCR, such as for example a mixture comprising dNTPs and a PCR reaction buffer, and optionally a DNA polymerase and probes suitable for PCR.

Method of MS-PCR

In a further aspect, the invention provides a method of methylation-specific polymerase chain reaction (MS-PCR), comprising amplifying bisulfite-treated DNA with one or more of the methylation-specific primers of the invention, one or more of the primer pairs of the invention, or one or more of the sets of the invention. All details of the corresponding aspects of the invention duly apply to the method of MS-PCR of the invention.

Methylation-specific polymerase chain reaction (MS-PCR) is a well-known technique for amplifying a DNA molecule to generate multiple copies of the DNA molecule based on its methylation pattern (Herman et al. (1996) Methylation-specific PCR: A novel PCR assay for methylation status of CpG islands. PNAS USA, 93:9821-9826; Derks et al. (2004) Methylation-specific PCR unraveled. Cellular Oncology, 26:291-299). As in any PCR, it relies on thermal cycling consisting of cycles of repeated heating and cooling of the reaction for DNA melting and enzymatic replication and amplification of the DNA.

Thus, typically the MS-PCR method of the invention comprises the steps:

- providing a DNA sample;
- treating the DNA sample with sodium bisulfite to convert the unmethylated cytosines in the DNA to uracil;
- amplifying the sodium bisulfite-converted DNA with one or more methylation-specific primers of the invention, one or more primer pairs of the invention, or one or more sets of the invention.

After or during the MS-PCR, amplification products of the PCR can be measured or detected using any suitable method in the art. Detection may occur in a separate step following amplification or, preferably, during amplification, for example using real-time MS-PCR. In a preferred embodiment, the MS-PCR method of the invention is a real-time MS-PCR method.

Detection or measurement can proceed in a semi-quantitative or a quantitative manner by real-time MSP, which provides high analytical sensitivity and a further improvement in lowering numbers of false-positive and false-negative cases (Kristensen LS and Hansen LL. (2009) PCR-based methods for detecting single-locus DNA methylation biomarkers in cancer diagnostics, prognostics, and response to treatment. Clin Chem, 55(8): 1471-83; Livak KJ and Schmittgen TD (2001) Methods; 25 (4): 402-8).

In a preferred embodiment, the MS-PCR method of the invention is a quantitative MS-PCR method, in particular a real-time methylation-specific PCR. In these methods, the quantification can be absolute or relative. Preferably, a relative quantification of DNA methylation is employed in the MS-PCR method of the invention, wherein the level of methylation measured for a certain target gene locus in a test sample is normalized to the level of methylation measured for an internal reference gene. The internal reference gene is typically selected from a region that does not contain any CpG nucleotides to allow an unbiased amplification. Any suitable reference gene can be used. Examples of reference genes are, for example, myoblast determination protein 1 (MYOD1), β actin (ACTB), or GAPDH, but any DNA sequence that does not contain any CpG dinucleotides and that is suitable for PCR amplification can be used. In one embodiment, the MS-PCR method of the invention is a quantitative, real-time PCR method and preferably further comprises:

- quantitatively determining the level of methylation of a target sequence in a DNA sample;
- quantitatively determining the level of methylation of a control sequence in the DNA sample; and
- normalizing the level of methylation of the target sequence to the level of methylation of the control sequence to obtain a normalized value of methylation of the DNA sample.

The level of methylation is used herein to refer to the degree of methylation, which in PCR methods, is indirectly determined by parameters of the PCR itself, e.g. the cycle threshold value, and the like. The parameter is not restricted, but the parameter selected to determine the level of methylation must be the same for all samples and sequences measured.

In another embodiment, the normalized value for the level of methylation of a "test" DNA sample is then calibrated to the normalized value for the level of methylation of a "control" DNA sample. In this embodiment, the normalized value for the level of methylation is quantitatively determined in a control DNA sample in the same manner as for the test sample, and the normalized level of methylation of the control sample is then subtracted from the normalized level of methylation of the test sample to obtain a normalized and calibrated value for the level of methylation of a test DNA sample. This is summarized in the comparative $C_T$ method, also referred to as the $2^{-\Delta\Delta Ct}$ method, which is a widely used method to present relative gene expression. The calculation is as follows:

$$2^{-\Delta\Delta Ct} = [(C_T \text{ gene of interest} - C_T \text{ internal control})\text{test}$$

$$- (C_T \text{ gene of interest} - C_T \text{ internal control})\text{control}$$

[Equation 1]

The control sample is not limited, but is typically a sample that is not suspected to show the alterations that are of interest in a test sample. For example, if the test sample is a pathological sample, such as a tumor tissue sample, the control sample could be, for example, a sample from a healthy control subject or pool thereof, or a sample taken from the same source (e.g. patient) as the pathological sample, but from a healthy specimen (e.g. non-tumor tissue). The control sample is not tumor-adjacent tissue.

In a particularly preferred embodiment, the MS-PCR method is an idiolocal normalized real-time methylation-specific PCR (Santourlidis et al. (2016) IDLN-MSP: Idiolocal normalization of real-time methylation-specific PCR for genetic imbalanced DNA specimens. Biotechniques, 60(2), pp.84-87). In an idiolocal normalized real-time methylation-specific PCR, the methylation-specific amplification level is normalized to an idiolocal locus, i.e. a locus near to the target sequence that does not contain any CpG dinucleotides, as described under the primer pair aspect of the invention. By selecting an idiolocal normalization reference instead of other references uncoupled to the DNA sequence of interest, numbers of both the methylation-specific and the unmethylated control sequences should be equal in almost every case for a given sample, which means that the amplification efficiencies of both targets will be comparable. When the methylation-specific amplification level is normalized to the idiolocal control, this gives a normalized methylation value that allows for reliable and comparable measurements even in DNA samples with varying numbers of the involved loci targeted by a real time MSP approach.

The DNA provided can be prepared from any biological sample or specimen that contains DNA, in particular that contains DNA comprising the DNA region of SEQ ID NO:1 and/or 6. The method of the invention is not limited to cellular DNA, but is also suitable for cell-free DNA. In a preferable embodiment, the DNA is isolated DNA, in particular genomic and/or eukaryotic DNA. In certain embodiments, the DNA is prepared from one or more cells, tissues, and/or body fluids. The cells and/or tissues can be of any type, or derived from any type. For example, the cells and/or tissues can be from brain, lung, skin, prostate, kidney, heart, prostate, or bladder tissues. In a particularly preferred embodiment, the DNA is prepared from a sample from the bladder, in particular a urothelium sample, and in particular from one or more cells or tissues from a urothelial sample. In another particularly preferred embodiment, the DNA is prepared from a sample from the prostate, in particular from one or more cells or tissues from a prostate sample. In certain embodiments, the DNA is prepared from one or more body fluids, preferably from a blood serum, blood plasma, urine, semen, lymph, cerebrospinal fluid, sputum, airway mucosa, and/or saliva sample. In a preferred embodiment, the DNA is prepared from a blood serum, blood plasma, or urine sample.

*In vitro* method of diagnosing, prognosing. and/or monitoring

In a further aspect, the invention provides an *in vitro* method for diagnosing, prognosing, and/or monitoring a tumor, comprising

a) determining the level of methylation of one or more CpG dinucleotides comprised in one of SEQ ID NO: 1-9 of a test sample; in particular in SEQ ID NO: 1 and/or 6 of the test sample;
b) determining the level of methylation of the same one or more CpG dinucleotides comprised in one of SEQ ID NO: 1-9 of a control sample; in particular in SEQ ID NO: 1 and/or 6 of the control sample;
c) comparing the level of methylation of the one or more CpG dinucleotides of the test sample to that of the control sample;

wherein a hypermethylation indicates the presence of a tumor, or indicates a high likelihood of developing a tumor; in particular of a urothelial bladder tumor or a prostate tumor.

The level of methylation can be determined using any standard technique. In one embodiment, the level of methylation is measured using genomic sequencing, in particular using bisulfite sequencing. Bisulfite sequencing of DNA is a well known technique in the art (e.g. Darst et al. (2010) Curr Protoc Mol Biol., CHAPTER: Unit-7.917) and in general comprises the steps of bisulfite conversion, molecular amplification and cloning, followed by genetic sequencing. The five basic steps in bisulfite conversion are: 1) DNA denaturation; 2) incubation with bisulfite at elevated temperature; 3) removal of bisulfite by desalting; 4) desulfonation of sulfonyl uracil adducts at alkaline pH; and 5) removal of the desulfonation solution. Following bisulfite conversion the templates are amplified. Typically it is recommended to perform three separate PCR reactions and, after pooling reactions, purify amplicon DNA, and then perform electrophoresis to quantify DNA yield digitally. Pooling multiple PCR reactions lessens the influence of stochastic events in early-stage amplification of individual template molecules.

If the level of methylation is determined by genomic sequencing, in particular by bisulfite sequencing, typically the number of CpG dinucleotides within a certain DNA region is counted for each sample. The number of CpG dinucleotides that are methylated is divided by the number of CpG dinucleotides that are comprised in the DNA region, which is then multiplied by 100 to give a certain percentage. Typically this is averaged over at least 5, preferably at least 10 runs. The authors found that a hypermethylation within SEQ ID NO: 1, and in particular within SEQ ID NO: 6 was indicative of a tumor, in particular of a bladder or a prostate tumor. In embodiments where the level of methylation is determined by genomic sequencing, in particular by bisulfite sequencing, and calculated by dividing the number of methylated CpG dinucldeotides over the total number of CpG dinucleotides comprised in SEQ ID NO: 1 and/or 6, a hypermethylation is defined to be an increase of 50% or more of the control sample. For example, if the control sample has an average level of methylation of 10%, a level of methylation of 15% or more would indicate a tumor. Preferably, in these methods, a hypermethylation is defined to be an increase of 100% or more of the control sample. For example, if the control sample has an average level of methylation of 10%, a level of methylation of 20% or more would indicate a tumor.

In another preferred embodiment, the level of methylation is measured using methylation-specific PCR, in particular the MS-PCR described in the above aspect of the invention. When calculating the level of hypermethylation with the MS-PCR method of the invention, preferably the comparative $C_T$ method, i.e. the $2^{-\Delta\Delta Ct}$ method is used.

When using methylation-specific PCR, and in particular the comparative $C_T$ method to calculate the level of hypermethylation, a hypermethylation is defined to be a level of methylation that is at least two times as high as the same in the normalized control sample. For example, a hypermethylation may be defined as being a level of methylation of two times or more, 5 times or more, 10 times or more, 20 times or more, 30 times or more, 40 times or more, 50 times or more, 100 times or more, or 150 times or more than the level of methylation of the same DNA sequence in a control sample. Preferably, a hypermethylation is defined as being a level of methylation of 10 times or more than the level of methylation of the control sample, preferably 20 times or more, 30 times or more, 40 times or more, or 50 times or more than the level of methylation of the DNA from a control sample. In a particularly preferred embodiment, a hypermethylation when using methylation-specific PCR is defined as being a level of methylation of 50 times or more than the level of methylation of the control sample, preferably 100 times or more, 150 times or more, 200 times or more, 300 times or more, 400 times or more, or 500 times or more than the level of methylation in a control sample. The levels of methylation should be normalized values when using methylation-specific PCR, preferably normalized to an idiolocal locus.

The invention also provides an *in vitro* method for diagnosing, prognosing, and/or monitoring a tumor, comprising

a) providing a test sample comprising or consisting of DNA;
b) treating the DNA with sodium bisulfite to convert the unmethylated cytosines to uracil;
c) amplifying the sodium bisulfite-converted DNA with one or more methylation-specific primers of the invention, one or more primer pairs of the invention, or one or more sets of the invention;
d) determining the level of methylation of the amplified sodium bisulfite-converted DNA;
e) repeating steps (a)-(d) for a control sample comprising or consisting of DNA;
f) comparing the level of methylation of the amplified sodium bisulfite-converted DNA from the test sample to the level of methylation of the amplified sodium bisulfite-converted DNA of the control sample, preferably using a comparative $C_T$ method;

wherein a hypermethylation in the test sample as compared to the control sample indicates the presence of a tumor, or indicates a high likelihood of developing a tumor; in particular of a bladder tumor or a prostate tumor.

In a preferred embodiment, a hypermethylation in the test sample as compared to the control sample indicates the presence of a urothelial bladder tumor, a prostate tumor tumor, or a tumor-adjacent tissue. In some embodiments, a hypermethylation indicates a high likelihood of developing a tumor, in particular a urothelial bladder tumor or a prostate tumor. Hypermethylation is defined as above for methods using methylation-specific PCR.

In certain embodiments, the test DNA is prepared from a sample taken from a patient suspected of having a tumor. Preferably, the test DNA is prepared from a suspected tumor tissue and/or a tumor-adjacent tissue, or is prepared from a tissue suspected to comprise tumor cells and/or tumor adjacent cells. In another embodiment, the DNA is prepared from a confirmed or suspected tumor tissue, or a confirmed or suspected tumor-adjacent tissue. The term "tumor-adjacent tissue" as used herein refers to a tissue that appears normal, e.g. histologically, and is adjacent to a pathologically classified or suspected tumor tissue. In certain embodiments, the DNA is prepared from one or more cells of a confirmed or suspected tumor or tumor-adjacent tissue. In a particularly preferred embodiment, the DNA is prepared from a tumor or tumor-adjacent sample from the bladder, in particular a tumor or tumor-adjacent urothelium sample, and in particular from one or more cells or tissues from a tumor or tumor-adjacent urothelial sample. In certain embodiments, the DNA is prepared from one or more body fluids, preferably from a blood serum, blood plasma, or urine from a patient with a suspected or confirmed tumor, in particular from a patient with a suspected or confirmed tumor of the bladder.

The control sample is typically a DNA sample that is not suspected to show the alterations that are of interest in a test sample. In certain embodiments, the DNA sample is prepared from healthy tissue of the same type from a healthy control subject or pool thereof, or a healthy tissue of the same type from the same source (e.g. patient) as the pathological sample. In a particularly preferred embodiment, the control sample is or is derived from healthy primary urothelium or healthy prostate tissue. The control sample is not tumor-adjacent tissue.

The inventors found that when the level of methylation is determined in bisulfite-converted DNA within SEQ ID NO: 1 and/or 6, in particular in bisulfite-converted DNA within the subregion SEQ ID NO: 6, partially methylated insert sequences derived from low grade urothelial tumor tissue were more than 150 times more highly methylated than the insert sequences derived from healthy primary urothelium. Densely methylated insert sequences from high grade urothelial tumor tissue were measured to be 70 times higher than even the partially methylated insert sequences derived from low grade tumor tissue. Thus this DNA region is highly sensitive to differential methylation in tumors and can be used to prognose, diagnose, and/or monitor the presence of a tumor in a patient. In one embodiment, a hypermethylation level of 150 times or more compared to a control sample, in particular healthy primary urothelium, indicates the presence of a high-grade tumor, in particular a high-grade urothelial tumor. Since not only tumor tissue, but also histologically normal tumor-adjacent tissue was found to have this altered methylation pattern, the method of the invention is also suitable for diagnosing, prognosing, and/or monitoring a tumor even from histologically normal appearing tissue.

Furthermore, since the method of the invention clearly differentiates partially methylated and densely methylated versions of sequences within SEQ ID NO: 1, in particular SEQ ID NO: 6, in particular using the methylation-specific primers of the invention, it is also suitable for stratifying patients and diagnosing, prognosing, and/or monitoring high grade tumors vs. low grade tumors, in particular in tumors of the bladder, such as low-grade and high-grade urothelial tumors, or in tumors of the prostate.

The above-described aspects of the invention will be further described in the following examples and figures. These are strictly illustrative of particularly preferred embodiments of the invention. The invention should not be construed, however, to be limited to such embodiments.

## EXAMPLES

### Example 1

Primary urothelium and the cell lines hREC (Normal Human Primary Renal Mixed Epithelial Cells, PCS-400-012, ATCC, Virginia, USA), HBLAK (Hoffmann, Michèle J., et al. "The new immortalized urothelial cell line HBLAK contains defined genetic aberrations typical of early stage urothelial tumors." Bladder Cancer 2.4 (2016): 449-463.), HEK293 (Frank. L. Graham et al.: Characteristics of a Human Cell Line Transformed by DNA from Human Adenovirus Type 5. In: Journal of General Virology,. Band 36, Nr. 1, 1977, S. 59-72), SW1710 (Kyriazis, Aikaterini A., et al. (1984) "Morphological, biological, and biochemical characteristics of human bladder transitional cell carcinomas grown in tissue culture and in nude mice." Cancer research 44(9): 3997-4005.) and HELA (Scherer, W. F., Syverton, J. T., & Gey, G. O. (1953). Studies on the propagation in vitro of poliomyelitis viruses: IV. Viral multiplication in a stable strain of human malignant epithelial cells (strain HeLa) derived from an epidermoid carcinoma of the cervix. The Journal of experimental medicine, 97(5), 695-710) were used to measure the expression of lncRNA *hTAPAS* and *HTERT* gene.

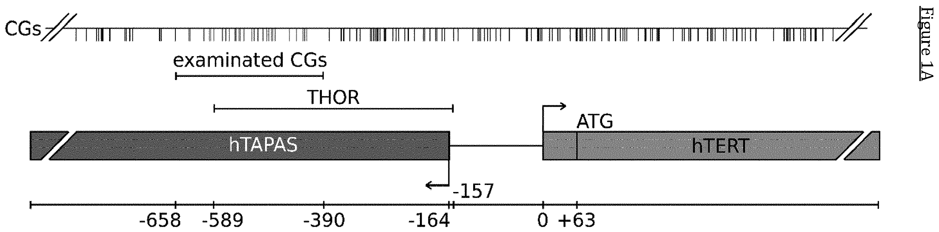

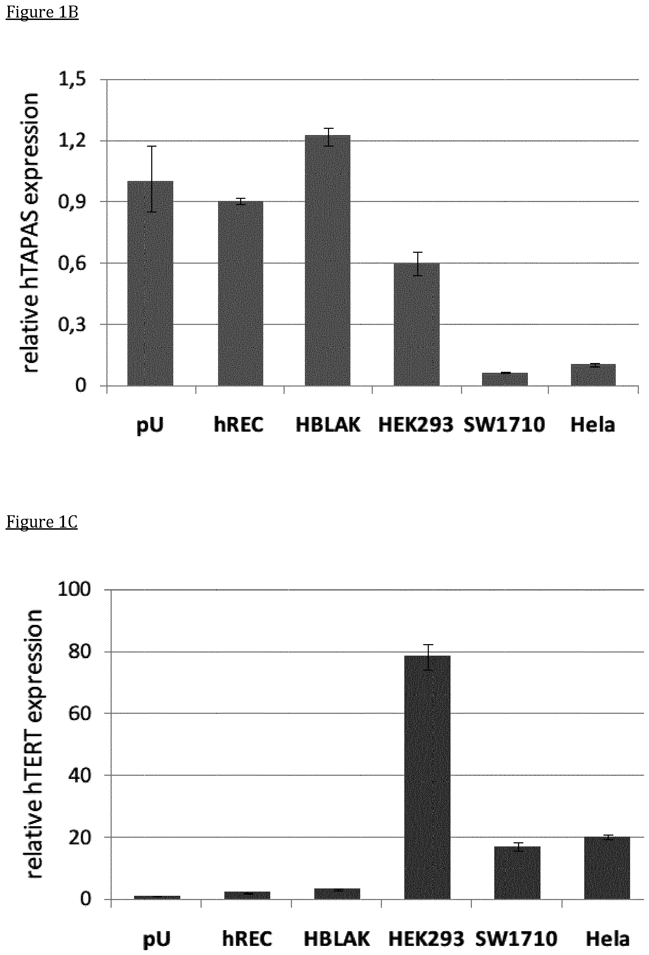

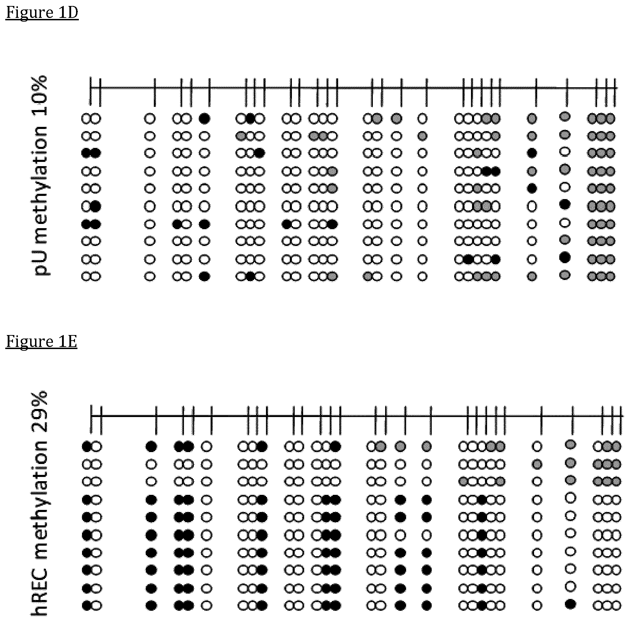

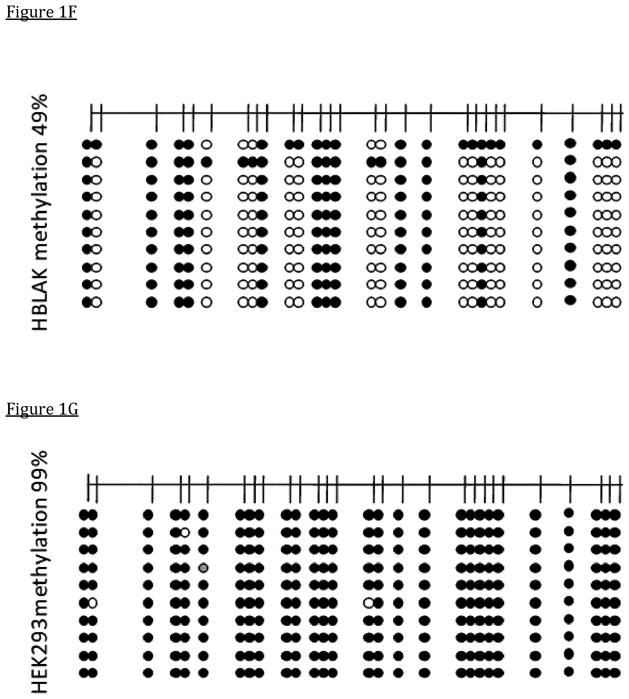

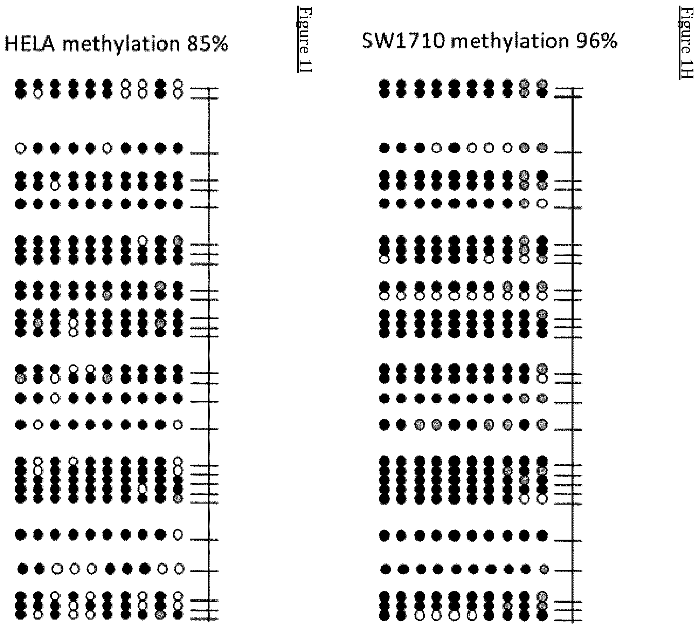

The results of the relative *hTAPAS* and *hTERT* expression in the various cell types are depicted in Figures 1B and 1C. The inventors found that primary urothelium (pU), primary renal epithelial cells (hREC) and the benign urothelial cell line HBLAK do not express *hTERT* at high levels, but show an increased *hTAPAS* expression by direct comparison to the adenovirus type 5 (Ad5) immortalized embryonic human kidney cell line HEK293, the urothelial carcinoma cell line SW1710 and the cervix carcinoma cell line HeLa. The HEK293, SW1710, and HeLa cell lines all had diminished *hTAPAS* expression but increased *hTERT* mRNA expression.

The respective DNA methylation profiles showed comparatively low methylation of SEQ ID NO: 6, namely 10% in pU, 29% in hREC, and 49% in HBLAK. In the latter two cell lines methylation follows an unusual pattern whereas certain CpGs were methylated while others were spared. In comparison HEK293, SW1710 and HeLa showed dense DNA methylation of 99%, 96% and 85% of this region, respectively. These results are depicted in Figures 1D to 11, wherein unfilled circles represent every unmethylated CpG dinucleotide, black filled circles represent every methylated CpG dinucleotide, and filled grey filled circles represent not defined CpG positions. The vertical lines represent each CpG dinucleotide comprised in SEQ ID NO: 6, i.e. CpG dinucleotides 23 to 50 from left to right in the figures.

These results show that DNA methylation of SEQ ID NO: 6 positively correlates with hTAPAS repression and hTERT expression in these cell types.

Example 2:

Genome-wide DNA methylation data sets were generated from pathologically reviewed and classified urothelial carcinoma and healthy urothelial tissue specimens by MeDIP and array analyses as described in Erichsen et al. (2018) Scientific Reports 8:3477. In brief, 4 groups of tissue specimen were compared to four samples of healthy primary urothelial tissue (pU). The other 4 groups of tissue samples consisted of 10 specimens of "UC" from unifocal papillary tumors (UT), 5 specimens adjacent to unifocal tumors, histologically verified as normal urothelium ("adjUT"), 5 tissue specimens of UC from multifocal tumors ("MT"), 5 specimens adjacent to multifocal tumors, histologically verified as normal urothelium ("adjMT").

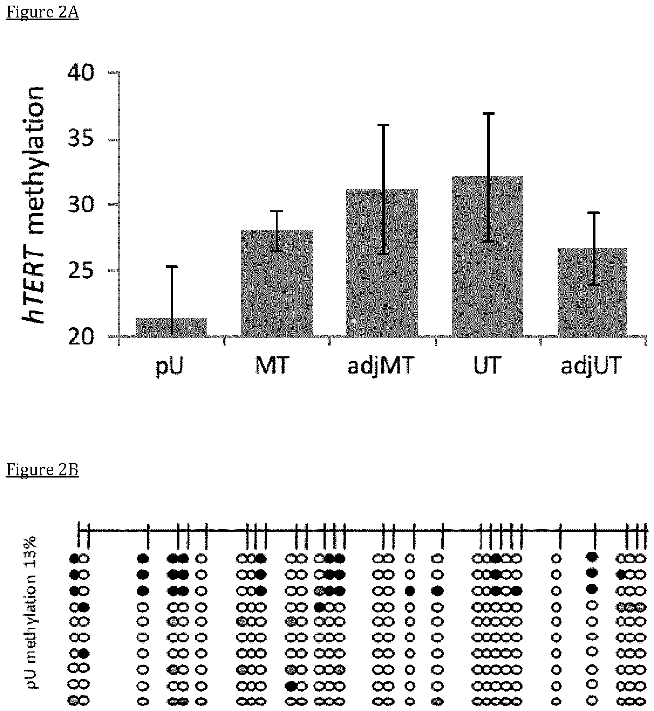

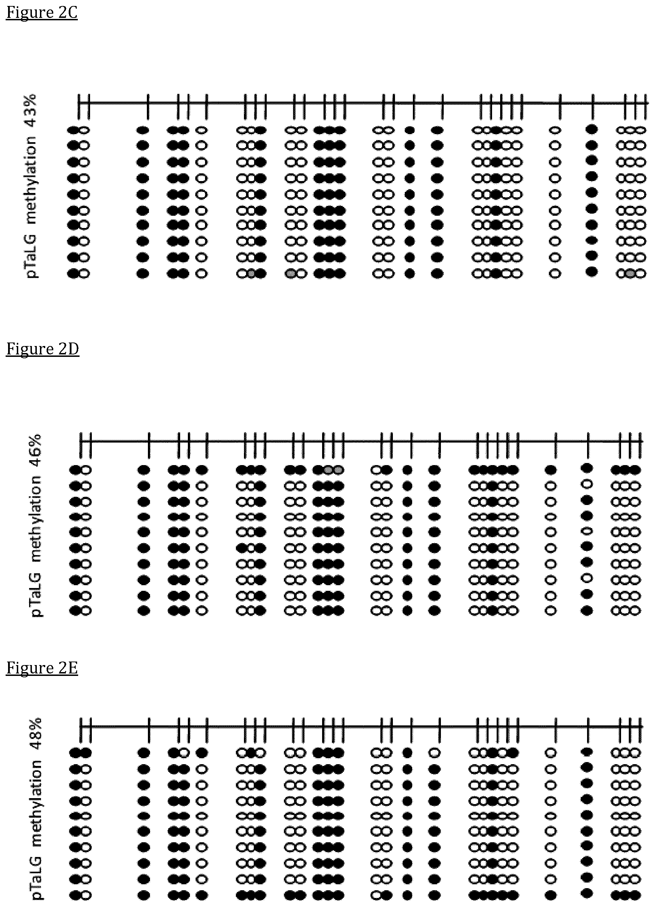

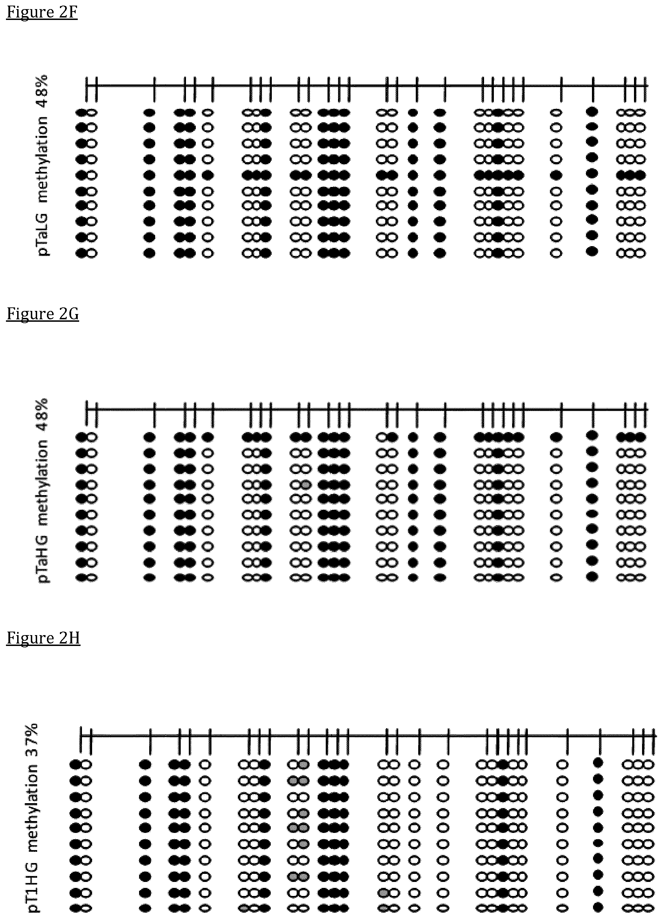

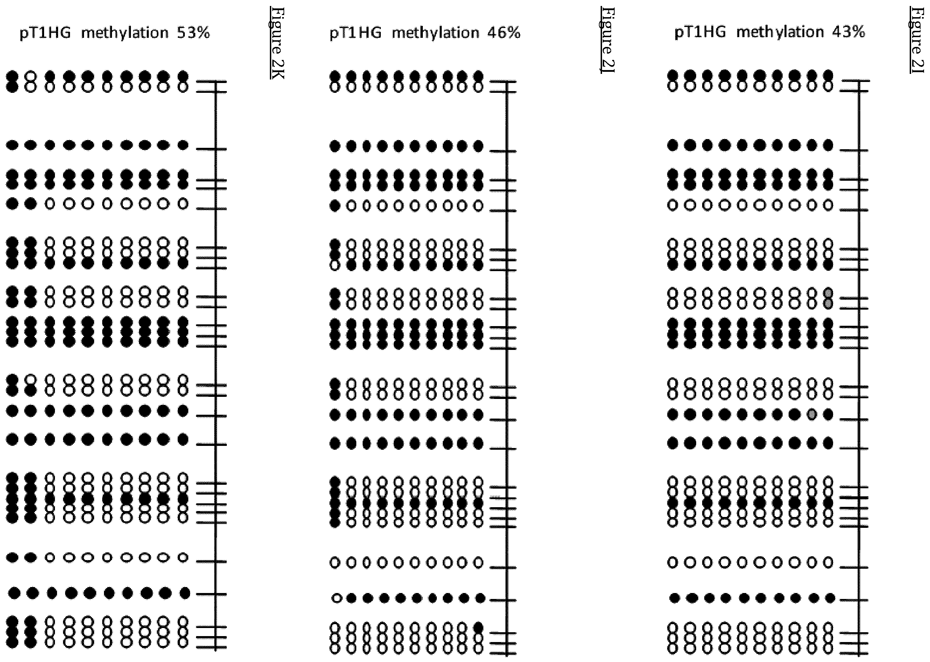

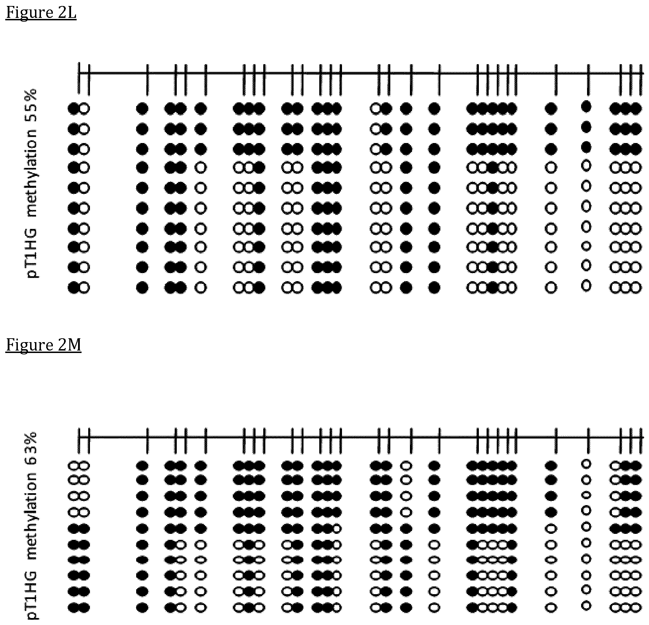

For each individual sample, integrated peak values for overall methylation of the relevant CpG-rich region direct upstream of the *hTERT* core promoter were obtained. Mean values of these peak values were calculated for each sample group. The statistical significance of differentially methylated regions (DMRs) was calculated for each group of samples compared to the healthy urothelium reference, based on the mean peak values by two sample Student's t-test with a significance threshold αDMR = 0.05. The relative degree of HTERT methylation detected by MSPCR is given in Figure 2A. Within the 5'-regulatory region of *hTERT,* in particular within SEQ ID NO: 6, a significant hypermethylation of was observed in MT, adj MT, UT, and adjUT, compared to pU (see Figure 2A).

Thereafter, detailed DNA methylation patterns in one pU specimen and eleven pTa and pT1 urothelial bladder cancer specimens were determined by bisulfite sequencing. The results are listed in Table 4 and depicted in Figure 2B to 2M.

**Table 4. Methylation of CpG dinucleotides in SEQ ID NO: 6 in bladder cancer patients**

| Sample | Average level of methylation | Sample | Average level of methylation |
|--------|------------------------------|--------|------------------------------|
| pU | 13% | pT1 - HG | 37% |
| pTa - LG | 43% | pT1 - HG | 43% |
| pTa - LG | 46% | pT1 - HG | 46% |
| pTa - LG | 48% | pT1 - HG | 53% |
| pTa - LG | 48% | pT1 - HG | 55% |
| pTa - HG | 48% | pT1 - HG | 63% |

Bisulfite sequencing was performed following bisulfite conversion with the EpiTec Kit (Qiagen, Hilden, Germany) as described (Santourlidis, S. et al. (2002) Crucial role of DNA methylation in determination of clonally distributed killer cell Ig-like receptor expression patterns in NK cells. The Journal of Immunology, 169(8):4253-4261; Ghanjati, F. et al. (2014) Unreserved application of epigenetic methods to define differences of DNA methylation between urinary cellular and cell-free DNA. Cancer Biomark. 14, 295-302).

In short, bisulfite conversion was performed using the EpiTect Kit (Qiagen) according to manufacturer's instructions. Primers were designed after excluding pseudogenes or other closely related genomic sequences which could interfere with specific amplification by amplicon and primer sequences comparison in BLAT sequence data base (https://genome.ucsc.edu/FAQ/FAQblat.html). The following forward and reverse primers were used:

| Primer Name | DNA sequence | SEQ ID NO: |
|---|---|---|
| s-HTERTPrkonv außen, 52°C | 5'- GGGTTTGTGTTAAGGAGTTTA -3' | 175 |
| as- HTERTPrkonv außen, 52°C | as: 5'- ACCATAATATAAAAACCCTAA -3' | 176 |

The pre-amplification conditions were denaturation at 95 °C for 13 min followed by 22 cycles of 95 °C for 50 s, TM for 45 s (50 °C), and 72 °C for 30 s. For the second round of the applied nested PCR the pre-amplification product was diluted 1:10 and the same PCR conditions were chosen for 35 additional cycles with the second pair of forward and reverse primers:

| Primer Name | DNA sequence | SEQ ID NO: |
|---|---|---|
| s-HTERTPrkonv innen, 52°C | 5'- GGTTTGTGTTAAGGAGTTTAAGT -3' | 177 |
| as- HTERTPrkonv innen, 52°C | 5'- atataaaaaccctaaaaacaaatac -3' | 178 |

The amplification products were 269 bp and 263 bp in size, respectively. Amplification products were cloned into pCR2.1 vector using the TA Cloning Kit (Invitrogen, Carlsbad, United States) according to the manufacturer's instructions.

Ten clones from each sample were sequenced using the BigDye Terminator Cycle Sequencing Kit (Applied Biosystems, Foster City, United States) on a DNA analyzer 3700 (Applied Biosystems) with M13 primer to obtain a representative methylation profile of each sample. 5'-regulatory gene sequences refer to +1 transcription start of the following sequences:

Homo sapiens telomerase reverse transcriptase (TERT) [gene], complete cds GenBank Reference Sequence: [AH007699]

Figures 2C to 2M depict the results in the form of comprehensive DNA methylation profiles of the THOR region from 4 pTa low grade (LG; Figures 2C to 2F), 1 pTa high grade (HG; Figure 2G) and 6 pT1-HG (Figures 2H to 2M) urothelial cancer samples. These profiles reveal a distinctive DNA methylation pattern in early urothelial cancers constituting of specific CpG dinucleotides which are consistently methylated while others remain largely unmethylated. In each of four pTaLG samples one sequence was methylated throughout while in three of the pT1HG samples the overall methylation level was increased due to 2-5 completely methylated sequences. In contrast, the same region was weakly methylated in primary urothelial samples (Fig. 1D and Fig. 2B).

Thus, the inventors found that hypermethylation of this CpG rich region nearby the *hTERT* core promoter occurs in early stage bladder (urothelial) cancer. A distinctive pattern of partial methylation predominates in pTa cancers which is maintained in pT1 tumors, but in some of these samples more densely methylated sequences were observed.

Example 3:

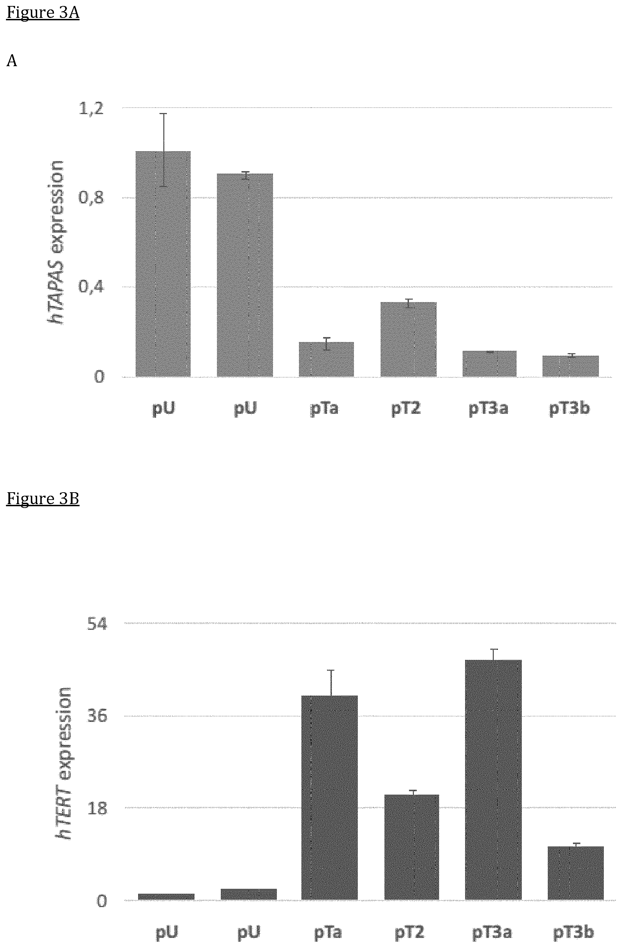

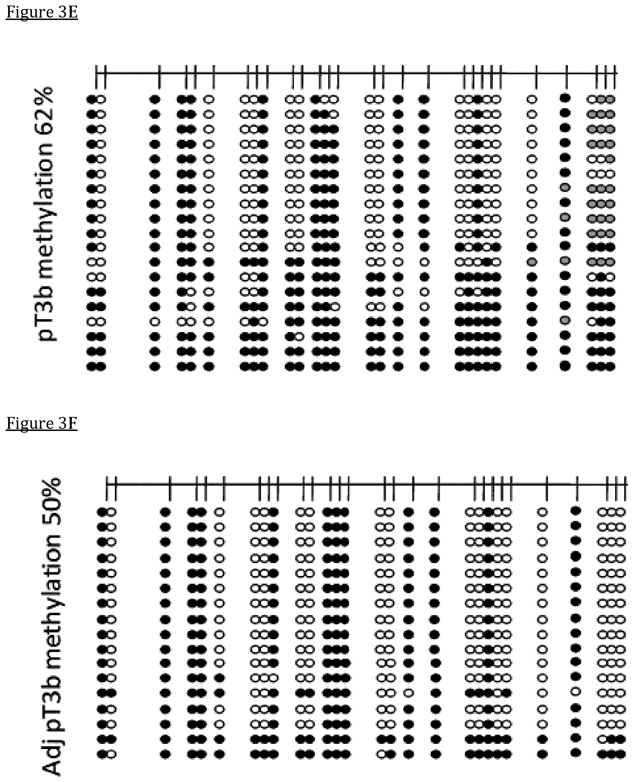

The expression levels of *hTAPAS* and *hTERT* were measured as described in Example 1, in one pTaLG, one pT2, one pT3a, one pT3b, and two primary urothelial (pU) tissue samples. The results are depicted in Figure 3A and 3B. In comparing the tumor tissue samples and the control (primary urothelial tissue) samples, the inventors found an inverse correlation between *hTAPAS* expression and *hTERT* expression.

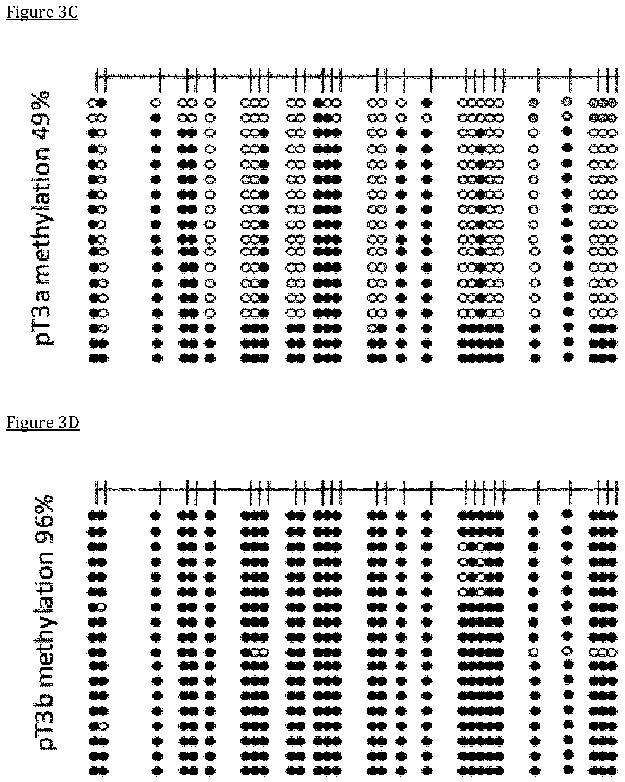

One pT3a and two pT3b microdissected tumor tissues together with one pT3b adjacent benign tissue samples were then analyzed in detail for their patterns of DNA methylation using bisulfite genome sequencing. The results are depicted in Figures 3C to 3F. The pT3a sample had a 49% level of methylation, the two pT3b samples had a 96% and a 62% level of methylation respectively, and the tumor-adjacent to the pT3b tumor showed a 50% level of methylation. The sequencing data reveal that in two cases the methylation profiles consist in part of the already known characteristic pattern with methylation affecting certain CpGs while sparing others and in part of completely methylated sequences. Interestingly, one pT3b sample, in contrast to all other cancer tissue samples, exhibited almost complete methylated sequences (96% methylation; Figure 3D) while the corresponding adjacent tissue showed the known partially methylated profile (see Figure 3F).

An essential, unresolved key point in the diagnosis of bladder cancer is to recognize in the early stage (pTa/pT1) whether the carcinoma will remain papillary or has the potential to grow muscle-invasively. The invention provides a means to identify tumor and tumor-adjacent tissue at an early stage, in particular in bladder cancer, which significantly improves the choice of the right therapy and will reduce morbidity and mortality.

Example 4:

A total of 27 plasmids were prepared as templates, all of which after sequencing were verified to bear the intended sequence of interest (in this case SEQ ID NO:6) prior to bisulfite conversion. 10 of the 27 plasmids were prepared from primary urothelium (pU). 9 of the 27 plasmids were prepared from papillar low grade tumor cells designed to comprise partially methylated SEQ ID NO: 6 inserts, and 8 of the 27 plasmids were designed to comprise enhanced methylated SEQ ID NO: 6 inserts.

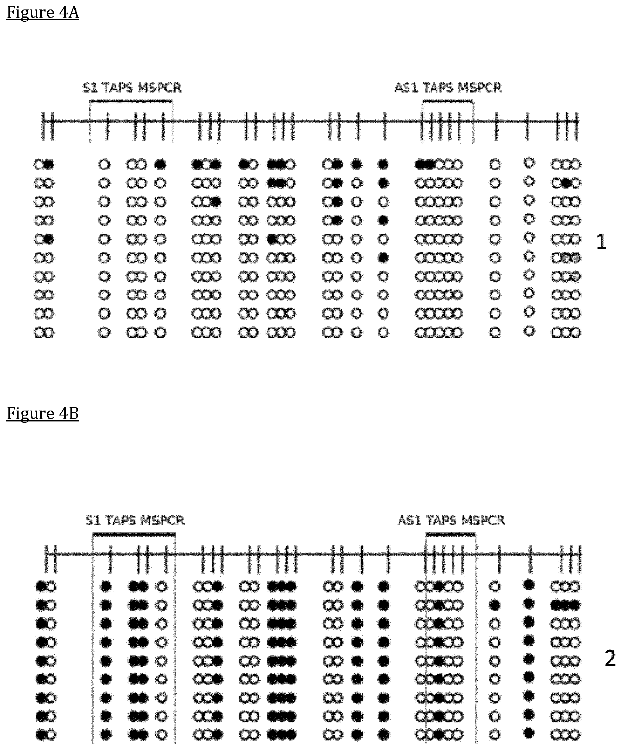

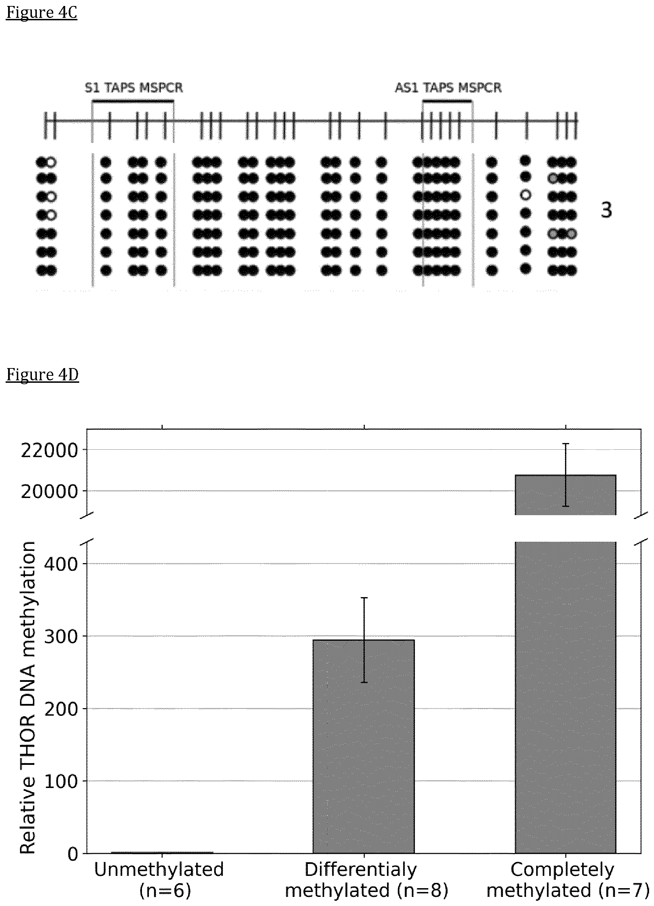

The results of bisulfite genomic sequencing are depicted in Figures 4A to 4C. Each row represents a single plasmid. Figure 4A represents the results from plasmids prepared from a primary urothelium (control) sample. Figure 4B represents the results from the 9 plasmids prepared from a pTa low grade tumor sample. Figure 4C represents the results from the 7 plasmids prepared from a pT1 high grade tumor sample.

The level of methylation in the respective plasmids was measured and quantified by idiolocal normalized methylation specific PCR as described in Santourlidis et al. (2016) BioTechniques 60:84-87. In detail, primers were carefully selected by OLIGO 4.1 Primer Analysis Software in order to have similar thermodynamic characteristics, i.e. TM, doublex free, hairpin free etc. and then per conditions were tested and adapted in order to obtain similar standard curves of the amplifications involved in a ΔCt calculation. In this context, an idiolocal (IDL) primer was selected to be as close as possible nearby the target amplification.

Real-time PCR was carried out with SYBR Green PCR Mastermix (Applied Biosystems) according to manufacturer's instructions. Per reaction 15 ng DNA, quantified by NanoDrop 1000 Spectrophotometer (Thermo Scientific, MA, USA), was converted by bisulfite and served as template for amplification of methylated sequences of interest in a normalized, real-time MSP using an idiolocal sequence (herein termed "IDL-TAPS"). The idiolocal sequence of interest used for normalization was located from -733 to -619 from AH007699.2 (115 bp). Primers were designed based on the previous data on differential methylated CpG patterns within SEQ ID NO: 6, in order to distinguish between hypo-, partial and dense methylated CpG positions within this region.

The primers used in this example are listed below. The methylation-specific primers used (SEQ ID NO: 189 and 190) are specific for the four CpG dinucleotides (bold, underlined): CpG dinucleotides 24, 25, 26, 27.

| Primer Name | DNA sequence | SEQ ID NO |
|---|---|---|
| Forward sense | 5'- TT**CG**GGAGGTTT**CGCG**TGTT**C** -3' | 179 |
| Reverse antisense | 5'- TCCAAATC**CG**AA**CGCG**AAA**CG** -3' | 180 |
| forward sense normalization (s-IDL-TAPS) | 5'- GAT TTG GGG TGG TTT GTT TAT G -3 | 181 |
| reverse antisense normalization (as-IDL-TAPS) | 5'- AAT ACC TCC CTA CAA CAC TTC CC -3' | 182 |

Relative template amounts were calculated following the $2^{-\Delta\Delta Ct}$-method with IDL-TAPS locus as the reference. SssI (New England Biolabs, Frankfurt am Main, Germany) *in vitro* methylated DNA of a primary uroepithelium has been used as calibrator.

Amplification conditions were denaturation at 95 °C for 10 min, followed by 40 cycles of 95 °C for 30 s, TM for 40 s, and 72 °C for 15 s. All real-time PCR and MS-PCR reactions were run in triplicate on a StepOnePlus System (Applied Biosystems, Foster City, CA). Standard curves were generated using StepOne software v2.1 (Applied Biosystems).

The plasmids were run individually and the results of the idiolocal normalized MS-PCR experiment were averaged and depicted in Figure 4D. The average and standard deviation for the unmethylated sequences was 0.97 ± 0.20, for partially methylated sequences 294.02 ± 58.30, and for densely methylated sequences was 20,763.23 ± 1,511.25. In summary, the hypomethylated insert sequences were measured to be more than 150 times lower methylated than the partially methylated insert sequences. The partially methylated insert sequences were measured to be 70 times lower than the enhanced (or "dense") insert methylated inserts.

Thus a clear differentiation of this differentially methylated genetic spot is feasible using the primers of the invention and provides a sensitive biomarker for diagnosing, prognosing, and monitoring cancer, in particular bladder cancer even in the early stages.

Example 5:

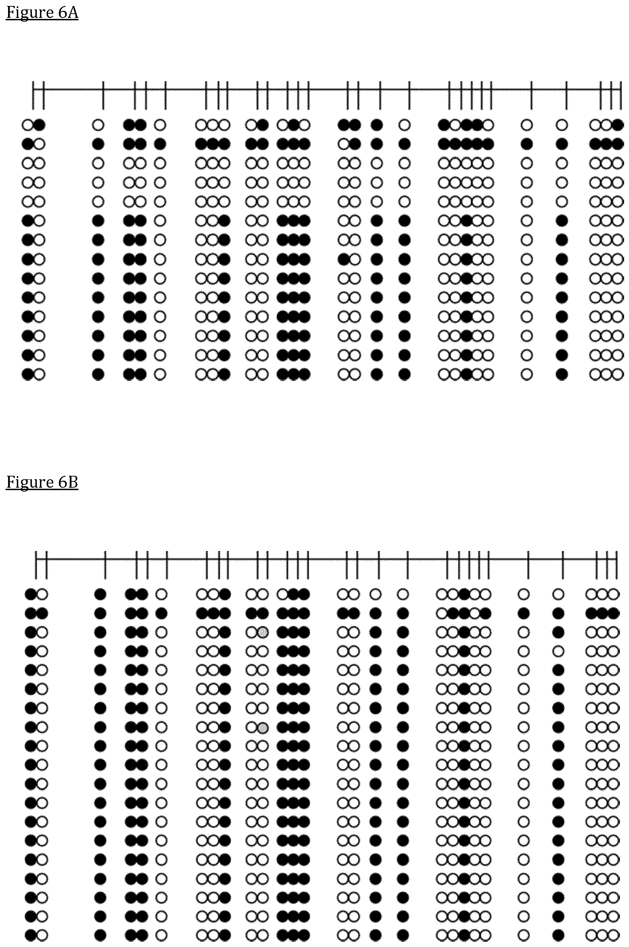

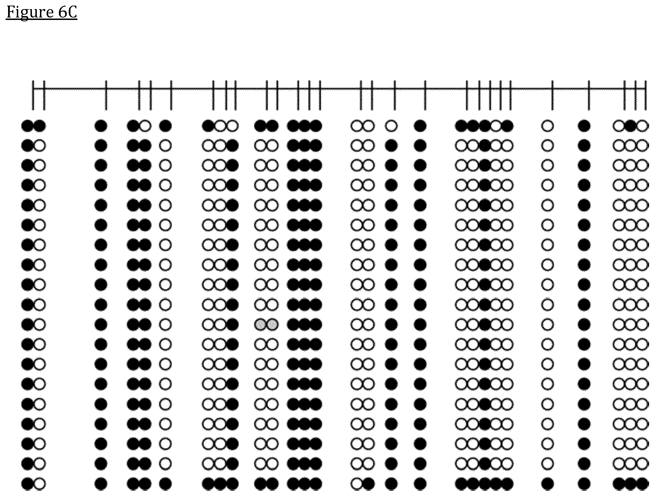

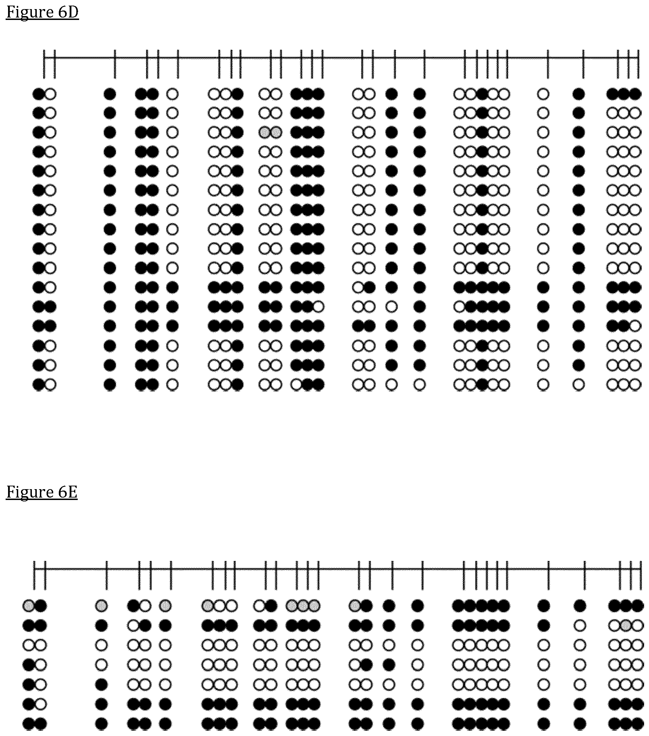

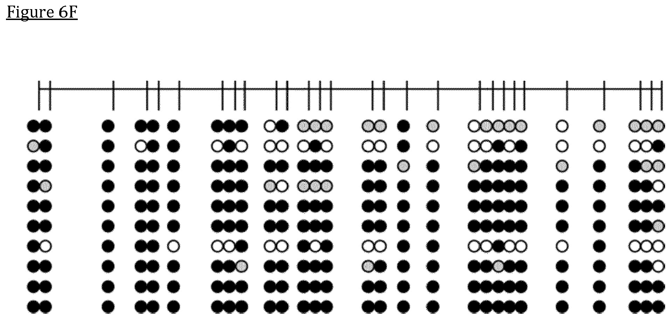

Five prostate tumor tissue samples (01-T to 05-T) together with one benign tumor-adjacent tissue (05-N) sample (taken from one of the same patients) were analyzed in detail for their patterns of DNA methylation within SEQ ID NO: 6 using bisulfite genome sequencing in the same manner as described for the bladder cancer samples. The results are depicted in Figure 6A-6F and listed in Table 5 below.

**Table 5. Methylation of CpG dinucleotides in SEQ ID NO: 6 in prostate cancer patients**

| Prostate sample ID | Tissue type | Clinical (Gleason) score | Average methylation of CpG dinucleotides in SEQ ID NO: 6 |
|---|---|---|---|
| 01-T | Prostate tumor | 3 + 3 = 6 | 38% |
| 02-T | Prostate tumor | 3 + 4 = 7 | 45% |
| 03-T | Prostate tumor | 3 + 3 = 6 | 47% |
| 04-T | Prostate tumor | 3 + 4 = 7 | 52% |
| 05-N | Normal tumor-adjacent | normal | 53% |
| 05-T | Prostate tumor | 3 + 4 = 7 | 84% |

The data in Table 5 and Figure 6A-F reveal that the hypermethylation status of the CpG dinucleotides comprised in SEQ ID NO: 1, and in particular those comprised in SEQ ID NO. 6, can provide a sensitive and reliable biomarker of prostate cancer, both in prostate tumor tissue and in prostate tumor-adjacent tissue, which seems otherwise healthy.

**BRIEF DESCRIPTION OF THE DRAWINGS**

Figure 1A depicts the exact location of the 269 bp 5'- fragment (see box with dotted line) ranging from position -658 to -390 relative to the transcription start site of *HTERT* gene, as well as the proportional distribution of the CpG dinucleotides within this genomic area relative to the *hTAPAS, THOR,* and the *hTERT* gene regions. Arrows indicate the transcription start site of the hTERT and hTAPAS genes.

Figure 1B depicts the results of the relative *hTAPAS* expression in various cell types. Primary urothelium (pU) and the cell lines hREC, HBLAK, HEK293, SW1710 and HELA were used to measure the expression of lncRNA *hTAPAS* gene.

Figure 1C depicts the results of the relative *hTERT* expression in various cell types. Primary urothelium (pU) and the cell lines hREC, HBLAK, HEK293, SW1710 and HELA were used to measure the expression of lncRNA *hTERT* gene.

Figure 1D depicts the detailed DNA methylation patterns measured in primary urothelium (pU) cells using bisulfite sequencing of SEQ ID NO: 6. Vertical lines at the top represent each CpG dinucleotide in SEQ ID NO: 6 (i.e. CpG numbers 23 to 50 as used herein). Unfilled circles represent every unmethylated CpG dinucleotide, black filled circles represent every methylated CpG dinucleotide, and filled grey filled circles represent not defined CpG positions.

Figure 1E depicts the detailed DNA methylation patterns measured in hREC cells using bisulfite sequencing of SEQ ID NO: 6. Vertical lines at the top represent each CpG dinucleotide in SEQ ID NO: 6. Unfilled circles represent every unmethylated CpG dinucleotide, black filled circles represent every methylated CpG dinucleotide, and filled grey filled circles represent not defined CpG positions.

Figure 1F depicts the detailed DNA methylation patterns measured in HBLAK cells using bisulfite sequencing of SEQ ID NO: 6. Vertical lines at the top represent each CpG dinucleotide in SEQ ID NO: 6. Unfilled circles represent every unmethylated CpG dinucleotide, black filled circles represent every methylated CpG dinucleotide, and filled grey filled circles represent not defined CpG positions.

Figure 1G depicts the detailed DNA methylation patterns measured in HEK293 cells using bisulfite sequencing of SEQ ID NO: 6. Vertical lines at the top represent each CpG dinucleotide in SEQ ID NO: 6. Unfilled circles represent every unmethylated CpG dinucleotide, black filled circles represent every methylated CpG dinucleotide, and filled grey filled circles represent not defined CpG positions.

Figure 1H depicts the detailed DNA methylation patterns measured in SW1710 cells using bisulfite sequencing of SEQ ID NO: 6. Vertical lines at the top represent each CpG dinucleotide in SEQ ID NO: 6. Unfilled circles represent every unmethylated CpG dinucleotide, black filled circles represent every methylated CpG dinucleotide, and filled grey filled circles represent not defined CpG positions.

Figure 1I depicts the detailed DNA methylation patterns measured in HELA cells using bisulfite sequencing of SEQ ID NO: 6. Vertical lines at the top represent each CpG dinucleotide in SEQ ID NO: 6. Unfilled circles represent every unmethylated CpG dinucleotide, black filled circles represent every methylated CpG dinucleotide, and filled grey filled circles represent not defined CpG positions.

Figure 2A depicts the mean of the peak values for overall hTERT methylation using array technology of SEQ ID NO: 1 from four samples of healthy primary urothelial tissue (pU) from a control subject, 10 specimens of urothelial bladder cancer ("UC") from unifocal papillary tumors ("UT"), 5 specimens adjacent to unifocal tumors, histologically verified as normal urothelium ("adjUT"), 5 tissue specimens of UC from multifocal tumors ("MT"), 5 specimens adjacent to multifocal tumors, histologically verified as normal urothelium ("adjMT").

Figures 2B-2M depict the detailed DNA methylation patterns in healthy primary urothelial tissue (pU; Figure 2B) or in urothelial bladder tumor specimens of grade Ta - low grade (Figures 2C - 2F), Ta - high grade (Figures 2G), or T1 - high grade (Figures 2H - 2M) that were determined by bisulfite sequencing of SEQ ID NO: 6. Vertical lines at the top represent each CpG dinucleotide in SEQ ID NO: 6 (i.e. CpG numbers 23 to 50 as used herein). Unfilled circles represent every unmethylated CpG dinucleotide, black filled circles represent every methylated CpG dinucleotide, and filled grey filled circles represent not defined CpG positions.

Figures 3A-3B depicts the expression levels of hTAPAS or hTERT in one pTaLG, one pT2, one pT3a, one pT3b, and two primary urothelial (pU) tissue samples.

Figures 3C-3F depict the patterns of DNA methylation using bisulfite genome sequencing from one pT3a (Fig. 3C) and two pT3b microdissected tumor tissues (Fig. 3D, 3E) together with one pT3b adjacent benign tissue sample (Fig. 3F).

Figures 4A-4C depict the results of bisulfite genomic sequencing from Experiment 4's plasmids prepared from a primary urothelium (control) sample (Figure 4A), from a pTa low grade tumor sample (Figure 4B), or from a pT1 high grade tumor sample (Figure 4C). Each row represents a single plasmid. Unfilled circles represent every unmethylated CpG dinucleotide, black filled circles represent every methylated CpG dinucleotide, and filled grey filled circles represent not defined CpG positions.

Figure 4D depicts the results of the idiolocal normalized MS-PCR experiment of Experiment 4.

Figure 5 depicts the numbering of the CpG dinucleotides in SEQ ID NO: 1.

Figure 6A shows the methylation status of the CpG dinucleotides of SEQ ID NO: 6 in a prostate cancer patient's tumor sample G072, which had a Gleason score of 3+3. There was average CpG dinucleotide methylation within this region of 38%.

Figure 6B shows the methylation status of the CpG dinucleotides of SEQ ID NO: 6 in a prostate cancer patient's tumor sample G106, which had a Gleason score of 3+4. There was average CpG dinucleotide methylation within this region of 45%.

Figure 6C shows the methylation status of the CpG dinucleotides of SEQ ID NO: 6 in a prostate cancer patient's tumor sample G110, which had a Gleason score of 3+3. There was average CpG dinucleotide methylation within this region of 47%.

Figure 6D shows the methylation status of the CpG dinucleotides of SEQ ID NO: 6 in a prostate cancer patient's tumor sample G192, which had a Gleason score of 3+4. There was average CpG dinucleotide methylation within this region of 52%.

Figure 6E shows the methylation status of the CpG dinucleotides of SEQ ID NO: 6 in a prostate cancer patient's normal tumor-adjacent tissue sample EP038N. There was average CpG dinucleotide methylation within this region of 53%.

Figure 6F shows the methylation status of the CpG dinucleotides of SEQ ID NO: 6 in tumor sample EPO38T taken from the same prostate cancer patient as measured in Figure 6E. This patient had a Gleason score of 3+4. There was average CpG dinucleotide methylation within this region of 84%.

**Claims**

1. A methylation-specific primer for amplifying one or more CpG dinucleotides comprised in SEQ ID NO: 1.

2. The methylation-specific primer according to claim 1, wherein the primer is specific for one or more CpG dinucleotides comprised in SEQ ID NO: 6.

3. The methylation-specific primer according to claims 1 or 2, wherein the primer is specific for two or more consecutive CpG dinucleotides comprised in SEQ ID NO: 1 and/or SEQ ID NO: 6; preferably for 2 or more, 3 or more, 4 or more, 5 or more, or 6 or more consecutive CpG dinucleotides comprised in SEQ ID NO: 1 and/or SEQ ID NO: 6.

4. The methylation-specific primer according to any one of the preceeding claims, wherein the one or more CpG dinucleotides are selected from the group consisting of CpG dinucleotide 28, 29, 30, 32, 33, 37, 38, 41, 42, 44, 45, or 46 of SEQ ID NO: 1; preferably for one or more CpG dinucleotides selected from the group consisting of CpG dinucleotides 28, 29, 30, 32, and 33 of SEQ ID NO: 1.

5. The methylation-specific primer according to any one of the preceeding claims, wherein the primer is specific for 2, 3, 4, or 5 consecutive CpG dinucleotides and wherein the CpG dinucleotides comprise or consist of CpG dinucleotide number 28 and one of its two adjacent CpG dinucleotides, CpG dinucleotide 29 and one of its two adjacent CpG dinucleotides, CpG dinucleotide 30 and one of its two adjacent CpG dinucleotides, CpG dinucleotide 32 and one of its two adjacent CpG dinucleotides, CpG dinucleotide 33 and one of its two adjacent CpG dinucleotides, CpG dinucleotide 37 and one of its two adjacent CpG dinucleotides, CpG dinucleotide 38 and one of its two adjacent CpG dinucleotides, CpG dinucleotide 41 and one of its two adjacent CpG dinucleotides, CpG dinucleotide 42 and one of its two adjacent CpG dinucleotides, CpG dinucleotide 44 and one of its two adjacent CpG dinucleotides, CpG dinucleotide 45 and one of its two adjacent CpG dinucleotides, or CpG dinucleotide 46 and one of its two adjacent CpG dinucleotides.

6. The methylation-specific primer according to any of the preceding claims, wherein the primer is from 18 to 35 bp in length; preferably from 20 to 30 bp in length.

7. The methylation-specific primer according to any of the preceding claims, wherein the primer is specific for a bisulfite-converted DNA sequence that has 90% or more, 95% or more, 98% or more, 99% or more, or 99.5% or more sequence identity to one of SEQ ID NO: 15 to 90 or SEQ ID NO: 179 for the sense strand and/or to one or SEQ ID NO: 91 to 174 or SEQ ID NO: 180 for the antisense strand; preferably to one of SEQ ID NO: 25 to 50 or 179 for the sense strand and/or to one of SEQ ID NO: 137 to 161 or 180 for the antisense strand; most preferably to SEQ ID NO: 179 for the sense strand and/or to SEQ ID NO: 180 for the antisense strand.

8. A methylation-specific primer pair for amplifying one or more CpG dinucleotides comprised in SEQ ID NO: 1, wherein the primer pair comprises the methylation-specific primer of any one of claims 1 to 7 and a primer that is methylation non-specific.

9. An *in vitro* assay or *in vitro* assay kit comprising the methylation-specific primer of any one of claims 1 to 7, or the primer pair of claim 8.

10. An *in vitro* method for diagnosing, prognosing, and/or monitoring a tumor, comprising

a) determining the level of methylation of one or more CpG dinucleotides comprised in one of SEQ ID NO: 1-9 of a test sample; in particular in SEQ ID NO: 6 of the test sample;
b) determining the level of methylation of the same one or more CpG dinucleotides comprised in one of SEQ ID NO: 1-9 of a control sample; in particular in SEQ ID NO: 6 of the control sample;
c) comparing the level of methylation of the one or more CpG dinucleotides of the test sample to that of the control sample;

wherein a hypermethylation in the one or more CpG dinucleotides of the test sample indicates the presence of a tumor, or indicates a high likelihood of developing a tumor.

11. An *in vitro* method for diagnosing, prognosing, and/or monitoring a tumor, comprising

(a) providing a test sample comprising or consisting of DNA;
(b) treating the DNA with sodium bisulfite to convert the unmethylated cytosines to uracil;
(c) amplifying the sodium bisulfite-converted DNA with one or more methylation-specific primers of any one of claims 1 to 7 or with one or more primer pairs of claim 8;
(d) determining the level of methylation of the amplified sodium bisulfite-converted DNA;
(e) repeating steps (a) to (d) for a control sample comprising or consisting of DNA;
(f) comparing the level of methylation of the amplified sodium bisulfite-converted DNA from the test sample to the level of methylation of the amplified sodium bisulfite-converted DNA from the control sample;

wherein a hypermethylation in the test sample as compared to the control sample indicates the presence of a tumor, or indicates a high likelihood of developing a tumor.

12. The *in vitro* method of claim 10 or 11, wherein the tumor is a urothelial bladder tumor or a prostate tumor.

13. The method of any one of claims 10 to 12, wherein a hypermethylation level is defined as being a level of methylation of two times or more, 5 times or more, 10 times or more, 20 times or more, 30 times or more, 40 times or more, 50 times or more, 100 times or more, or 150 times or more than the level of methylation of the DNA from the control sample; preferably a level of methylation of 50 times or more, 100 times or more, or 150 times or more than the level of methylation in the control sample.

14. The method of any one of claims 10 to 13, wherein a level of methylation of 300 times or more, 400 times or more, or 500 times or more than the level of methylation in the control sample indicates the presence of a high-grade tumor, in particular a high-grade urothelium tumor.

15. The method according to any one of claims 10 to 14, wherein the level of methylation is determined using quantitative real-time methylation-specific PCR, in particular using idiolocal normalized real-time methylation specific PCR.

16. Use of one or more of SEQ ID NO: 1-9 in an *in vitro* method of diagnosing, prognosing, and/or monitoring cancer; in particular a bladder or prostate cancer.

Figure 1A

Figure 1B

Figure 1C

Figure 1D

Figure 1E

Figure 1F

Figure 1G

Figure 1H

SW1710 methylation 96%

Figure 1I

HELA methylation 85%

Figure 2A

Figure 2B

Figure 2C

Figure 2D

Figure 2E

Figure 2F

Figure 2G

Figure 2H

Figure 2I

Figure 2J

Figure 2K

Figure 2L

Figure 2M

Figure 3A

A

Figure 3B

Figure 3C

Figure 3D

Figure 3E

pT3b methylation 62%

Figure 3F

Adj pT3b methylation 50%

Figure 4A

Figure 4B

Figure 4C

Figure 4D

Figure 5

EP 4 289 971 A1

gggaagtcctcagctgtcctg**CG**gttgtgc**CG**gggcccccaggtctggaggggaccagtggc**CG**tgtggcttctactgctgggctgga

agt**CG**ggcctcctagctctgcagtc**CG**aggcttggagccaggtgcctggaccc**CG**aggctgccctccaccctgtg**CG**gg**CG**ggat

gtgaccagatgttggcctcatctgccagacagagtgc**CG**gggcccagggtcaaggc**CG**ttgtggctggtgtgagg**CG**cc**CG**gtg**C**

**GCG**gccagcaggag**CG**cctggctccatttcccacccttct**CG**a**CG**ggac**CG**ccc**CG**gtgggtgattaacagatttgggggtggttt

gctcatggtggggacccct**CG**c**CG**cctgagaacctgcaaagagaaatga**CG**ggcctgtgtcaaggagcccaagt**CGCG**gggaa

gtgttgcagggaggcactc**CG**ggaggtcc**CGCG**tgcc**CG**tccagggagcaatg**CG**tcct**CG**ggtt**CG**tccccagc**CGCG**tct

a**CGCG**cctc**CG**tcctcccctca**CG**tc**CG**gcatt**CG**tggtgcc**CG**gagcc**CG**a**CG**ccc**CGCG**tc**CG**gacctggaggcagc

cctgggtctc**CG**gatcaggccag**CG**gccaaagggt**CG**c**CG**ca**CG**cacctgttcccagggcctccacatcatggcccctccct**CG**

ggttaccccacagcctaggc**CG**att**CG**acctctctc**CG**ctggggccct**CG**ctgg**CG**tccctgcaccctgggag**CGCG**ag**CG**g**C**

**GCGCG**gg**CG**gggaag**CGCG**gcccagacccc**CG**ggtc**CG**cc**CG**gagcagctg**CG**ctgt**CG**gggccaggc**CG**ggctcc

cagtggatt**CGCG**ggcacaga**CG**cccaggac**CGCG**ctcccca**CG**tgg**CG**gagggactggggacc**CG**ggcacc**CG**tcctg

ccccttcaccttccagctc**CG**cctcctc**CGCGCG**gaccccgccc**CG**tcc**CG**acccctcc**CG**ggtccc**CG**gcccagccccctc**C**

**G**ggccctcccagcccctccccttccttc**CGCG**gccc**CG**ccctctcct**CGCG**g**CGCG**agtttcaggcag**CG**ctg**CG**tcctgctg

**CG**ca**CG**tgggaagccctggcccc

Figure 6A

Figure 6B

Figure 6C

Figure 6D

Figure 6E

Figure 6F

**EP 4 289 971 A1**

Application Number

EP 22 17 7666

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 10 597 730 B2 (HOSPITAL FOR SICK CHILDREN [CA]) 24 March 2020 (2020-03-24) * the whole document * | 1-16 | INV. C12Q1/6886 |
| X | LEE DONGHYUN D. ET AL: "Dual role of allele-specific DNA hypermethylation within the TERT promoter in cancer", JOURNAL OF CLINICAL INVESTIGATION, vol. 131, no. 21, 1 November 2021 (2021-11-01), XP93001121, DOI: 10.1172/JCI146915 * the whole document * | 1-16 | |
| X | LEE DONGHYUN D ET AL: "DNA methylation of the TERT promoter and its impact on human cancer", CURRENT OPINION IN GENETICS & DEVELOPMENT., vol. 60, 1 February 2020 (2020-02-01), pages 17-24, XP93001128, XX ISSN: 0959-437X, DOI: 10.1016/j.gde.2020.02.003 * the whole document * | 1-16 | |
| X | RICARDO LEÃO ET AL: "Combined genetic and epigenetic alterations of the TERT promoter affect clinical and biological behavior of bladder cancer", INTERNATIONAL JOURNAL OF CANCER, JOHN WILEY & SONS, INC, US, vol. 144, no. 7, 30 December 2018 (2018-12-30), pages 1676-1684, XP071290663, ISSN: 0020-7136, DOI: 10.1002/IJC.31935 * the whole document * | 1-16 | |

TECHNICAL FIELDS SEARCHED (IPC)

C12Q

-/--

The present search report has been drawn up for all claims

| Place of search | Date of completion of the search | Examiner |
|---|---|---|
| The Hague | 24 November 2022 | Botz, Jürgen |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## EUROPEAN SEARCH REPORT

Application Number

EP 22 17 7666

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | MALHOTRA SANANDAN ET AL: "A Novel Long Non-Coding RNA in the hTERT Promoter Region Regulates hTERT Expression", NON-CODING RNA, vol. 4, no. 1, 29 December 2017 (2017-12-29), page 1, XP93001148, DOI: 10.3390/ncrna4010001 * the whole document * | 1-16 | |

-----

**TECHNICAL FIELDS SEARCHED     (IPC)**

The present search report has been drawn up for all claims

| Place of search | Date of completion of the search | Examiner |
|---|---|---|
| The Hague | 24 November 2022 | Botz, Jürgen |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 17 7666

This annex lists the patent family members relating to the patent documents cited in the above-mentioned European search report.
The members are as contained in the European Patent Office EDP file on
The European Patent Office is in no way liable for these particulars which are merely given for the purpose of information.

24-11-2022

| Patent document cited in search report | Publication date | Patent family member(s) | Publication date |
|---|---|---|---|
| US 10597730 B2 | 24-03-2020 | NONE | |

EPO FORM P0459

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **LEE DD et al.** DNA hypermethylation within TERT promoter upregulates TERT expression in cancer. *The Journal of clinical investigation,* 2019, vol. 129, 223-229
- **MALHOTRA S et al.** A Novel Long Non-Coding RNA in the hTERT Promoter Region Regulates hTERT Expression. *Non-coding RNA,* 2017, vol. 4, 10
- **CASTELO-BRANCO et al.** Methylation of the TERT promoter and risk stratification of childhood brain tumours: an integrative genomic and molecular study. *Lancet Oncol.,* 2013, vol. 14, 534-42
- **CASTELO-BRANCO et al.** A Cancer Specific Hypermethylation Signature of the TERT Promoter Predicts Biochemical Relapse in Prostate Cancer: A Retrospective Cohort Study. *Oncotarget,* 2016, vol. 7, 57726-57736
- **LEÃO R et al.** Combined genetic and epigenetic alterations of the TERT promoter affect clinical and biological behavior of bladder cancer. *Int J Cancer,* 2019, vol. 144 (7), 1676-1684
- **FALEIRO I et al.** The TERT hypermethylated oncologic region predicts recurrence and survival in pancreatic cancer. *Future Oncol,* 2017, vol. 13 (23), 2045-2051
- **CASTELO-BRANCO et al.** Methylation of the TERT promoter and risk stratification of childhood brain tumours: an integrative genomic and molecular study. *Lancet Oncol.,* 2013, vol. 14, 534-42
- **QURESHI SA ; BASHIR MU ; YAQINUDDIN A.** Utility of DNA methylation markers for diagnosing cancer. *Int J Surg.,* 2010, vol. 8 (3), 194-8
- **SIDRANSKY D.** Emerging molecular markers of cancer. *Nat Rev Cancer.,* 2002, vol. 2 (3), 210-9
- **HERMAN et al.** Methylation-specific PCR: A novel PCR assay for methylation status of CpG islands. *PNAS USA,* 1996, vol. 93, 9821-9826
- **WANG, R. Y.-H. ; GEHRKE, C. W. ; EHRLICH, M.** *Nucleic Acids Res.,* 1980, vol. 8, 4777-4790
- **DERKS et al.** Methylation-specific PCR unraveled. *Cellular Oncology,* 2004, vol. 26, 291-299
- **L.C. LI ; R. DAHIYA.** MethPrimer: designing primers for methylation PCRs. *Bioinformatics,* 2002, vol. 18, 1427-1431
- **ROZEN ; H. SKALETSKY.** Primer3 on the WWW for general users and for biologist programmers. *Methods Mol. Biol.,* 2000, vol. 132, 365-386

- **SANTOURLIDIS et al.** IDLN-MSP: Idiolocal normalization of real-time methylation-specific PCR for genetic imbalanced DNA specimens. *Biotechniques.,* 2016, vol. 60 (2), 84-7
- **KRISTENSEN LS ; HANSEN LL.** PCR-based methods for detecting single-locus DNA methylation biomarkers in cancer diagnostics, prognostics, and response to treatment. *Clin Chem,* 2009, vol. 55 (8), 1471-83
- **LIVAK KJ ; SCHMITTGEN TD.** *Methods,* 2001, vol. 25 (4), 402-8
- **SANTOURLIDIS et al.** IDLN-MSP: Idiolocal normalization of real-time methylation-specific PCR for genetic imbalanced DNA specimens. *Biotechniques,* 2016, vol. 60 (2), 84-87
- **DARST et al.** Curr Protoc Mol Biol. 2010
- **HOFFMANN, MICHÈLE J. et al.** The new immortalized urothelial cell line HBLAK contains defined genetic aberrations typical of early stage urothelial tumors. *Bladder Cancer,* 2016, vol. 2 (4), 449-463
- **FRANK. L. GRAHAM et al.** Characteristics of a Human Cell Line Transformed by DNA from Human Adenovirus Type 5. *Journal of General Virology,* 1977, vol. 36 (1), 59-72
- **KYRIAZIS, AIKATERINI A. et al.** Morphological, biological, and biochemical characteristics of human bladder transitional cell carcinomas grown in tissue culture and in nude mice. *Cancer research,* 1984, vol. 44 (9), 3997-4005
- **SCHERER, W. F. ; SYVERTON, J. T. ; GEY, G. O.** Studies on the propagation in vitro of poliomyelitis viruses: IV. Viral multiplication in a stable strain of human malignant epithelial cells (strain HeLa) derived from an epidermoid carcinoma of the cervix. *The Journal of experimental medicine,* 1953, vol. 97 (5), 695-710
- **ERICHSEN et al.** *Scientific Reports,* 2018, vol. 8, 3477
- **SANTOURLIDIS, S. et al.** Crucial role of DNA methylation in determination of clonally distributed killer cell Ig-like receptor expression patterns in NK cells. *The Journal of Immunology,* 2002, vol. 169 (8), 4253-4261
- **GHANJATI, F. et al.** Unreserved application of epigenetic methods to define differences of DNA methylation between urinary cellular and cell-free DNA. *Cancer Biomark.,* 2014, vol. 14, 295-302

- **SANTOURLIDIS et al.** *BioTechniques,* 2016, vol. 60, 84-87